# EUROPEAN PATENT APPLICATION

(11) **EP 1 004 596 A1**
(43) Date of publication of application: **31.05.2000**
(21) Application number: 98936718.0
(22) Date of filing: 10.08.1998
(51) Int. Cl.: C07K 14/47, C12P 21/02, C12N 15/12, C12Q 1/68, C07K 16/18

(54) **1(3)mbt PROTEIN, POLYNUCLEOTIDE CODING FOR SAID PROTEIN, ANTISENSE POLYNUCLEOTIDE THEREOF, AND ANTIBODY RECOGNIZING SAID PROTEIN**

(30) Priority: 13.08.1997 JP 23182797
(71) Applicant: Sumitomo Electric Industries, Ltd., Osaka-shi, Osaka 541-0041 (JP)
(72) Inventor: KISHIMOTO, Toshihiko;-Yokohama Works of Sumitomo E, Kanagawa 244-8588 (JP); NIWA, Shin-ichiro;-Yokohama Works of Sumitomo Elec, Kanagawa 244-8588 (JP); NAGAMINE, Yasuko;-Yokohama Works of Sumitomo Elect, Kanagawa 244-8588 (JP); KOGA, Hisashi, Kumamoto 860-0812 (JP); SAYA, Hideyuki, Kumamoto 862-0970 (JP)
(74) Representative: Baldock, Sharon Claire
(86) International application number: JP9803551
(87) International publication number: WO9909060

(57) **Abstract**

A gene homologous to a d-l(3)mbt gene (i.e. a homologous gene) is isolated, the base sequence thereof is determined, a protein coding for the gene (i.e. a homologous protein) is prepared, and an antibody recognizing the protein is harvested.

## Description

### Technical Field

This invention relates to homologous proteins of *drosophila* l(3)mbt protein and homologues of polynucleotide encoding said proteins. This invention further relates to antisense polynucleotides complementary to polynuceotides and antibodies recognizing said homologous proteins.

### Background Art

The nervous system of human beings is the most highly differentiated one among those of vertebrate. However, the analysis of the human nervous system is retarded due to its complicated mechanisms. In particular, progress of analysis of tumors developing in the cranial nerve system is slower than the analysis of other tumors. It is not only malignant tumors developing in the cranial nerve system that jeopardize the patient's life but also benign tumors may jeopardize the patient's life depending on the location at which the tumors develop. Therefore, improved understanding of the developmental mechanisms of cranial nerve tumors is desired.

On the other hand, many mutants have been obtained in *drosophila* during the analysis process of neural development, and genetic analysis thereof has been carried out. *Drosophila* l(3)mbt protein (referred to as d-l(3)mbt protein hereinafter) encoded by *drosophila* l(3)mbt gene (referred to as d-l(3)mbt gene hereinafter) has proline-rich domain and zinc finger domain and is a protein containing three portions of a mbt-repeat sequence composed of about 100 characteristic amino acids. Further, the deletion of d-l(3)mbt gene is known as the cause of the generation of malignant tumors of adult optic neuroblast and ganglion mother cell and the excessive proliferation of imaginal discs.

### Disclosure of Invention

An Object of the present invention is to search and isolate homologous genes of d-l(3)mbt gene, determine base sequences of said genes, prepare proteins encoded by said genes, and obtain antibodies recognizing said proteins.

Specifically, according to the present invention, a base sequence having homology to d-l(3)mbt is selected from EST database on the basis of the base sequence information of d-l(3)mbt gene, and a primer comprising a part of the selected base sequence is prepared. Further, by using this primer, a homologous gene in human beings (referred to as h-l(3)mbt gene hereinafter) is isolated by means of PCR using a cDNA library from human fetal brain as a template, and the base sequence thereof is disclosed.

Further, in the manner similar to above-described one, the present invention uses the primer in the isolation process of h-l(3)mbt gene and a primer newly prepared from a part of the above-selected base sequence to isolate a homologous gene of d-l(3)mbt gene in a mouse (referred to as m-l(3)mbt hereinafter) by means of PCR using a cDNA library from neonatal mouse brain as a template, and the base sequence of the m-l(3)mbt gene is disclosed.

Furthermore, in the manner similar to that described above, the present inventions isolates a homologous gene of d-l(3)mbt gene in a rat (referred to as r-l(3)mbt gene hereinafter), and discloses the base sequence thereof.

The base sequences of h-l(3)mbt gene, m-l(3)mbt gene and r-l(3)mbt gene have high homologies in the mbt-repeat portions.

The present invention discloses a method for acquiring homologous genes of d-l(3)mbt gene, h-l(3)mbt gene, m-l(3)mbt gene or r-l(3)mbt gene in other animals.

Specifically, a gene homologous to d-l(3)mbt gene in an animal other than human beings, mice or rats may be obtained by using a cDNA fragement obtained from the base sequence of h-l(3)mbt gene, m-l(3)mbt gene or r-l(3)mbt gene, or polynucleotide (for example, a single-stranded DNA, RNA complementary to cDNA or their chemically modified forms) obtained from the base sequence of said CDNA fragment as a probe to perform the hybridization using a cDNA library of the animal. Further, the homologous gene of d-l(3)mbt gene in the animal can be obtained by amplifying that in the cDNA library with PCR .

In this specification, the genes which are an individual elements in an aggregation of h-l(3)mbt gene, m-l(3)mbt gene, r-l(3)mbt gene and their homologous genes are referred to as l(3)mbt genes.

Further, the present invention provides a protein encoded by the above-stated 1(3)mbt gene. This protein is homologous to d-l(3)mbt, h-l(3)mbt, m-l(3)mbt gene or r-l(3)mbt. In this specification, a protein which is an individual element in an aggregation of h-l(3)mbt, m-l(3)mbt, r-l(3)mbt and their homologous proteins is referred to as l(3)mbt protein or merely l(3)mbt.

Furthermore, the present invention also discloses antisense polynucleotides comprising base sequences complementary to the above-mentioned base sequences.

Furthermore, the present invention provides a preparation method of l(3)mbt using a transformant with the l(3)mbt gene introduced therein.

Furthermore, the present invention shows the possibility of preparing an antibodies recognizing the l(3)mbt, and preparing antigenicity of the l(3)mbt, that is in general, antibodies from 1(3)mbt. Further, according to the present invention, l(3)mbt can be detected from animal tissues or animal cell strains on the basis of analysis by means of western blotting or ELISA using the above-mentioned antibodies.

Further, according to the present invention, mRNA of 1(3)mbt can be detected from various tissues and cell strains of human beings and mice on the basis of the analysis by means of northern blotting hybridization using a gene probe, and the present invention discloses the natural expression of mRNA of l(3)mbt in these animals.

Furthermore, the present invention discloses the location of h-l(3)mbt gene on chromosome.

### Brief Description of Drawings

Fig. 1 shows the comparison of the amino acid sequences of h-l(3)mbt, m-l(3)mbt and r-l(3)mbt.
Fig. 2 shows the comparison of the amino acid sequences of h-l(3)mbt and d-l(3)mbt.
Fig. 3 shows the location relationship of EST and cDNA fragments obtained in a process to acquire h-l(3)mbt gene.
Fig. 4 shows the location relationship of EST and cDNA fragments obtained in a process to acquire m-l(3)mbt gene.
Fig. 5 is a photograph of electrophoresis showing the results of northern blotting hybridization where cDNA of h-l(3)mbt type 1 is hybridized to mRNAs of various human tissues.
Fig. 6 is a photograph of electrophoresis showing the results of northern blotting hybridization where cDNA of h-l(3)mbt type 1 is hybridized to mRNAs of various human tissues.
Fig. 7 is a photograph of electrophoresis showing the results of northern blotting hybridization where cDNA of h-l(3)mbt type 1 is hybridized to mRNAs of various human tissues.
Fig. 8 is a photograph of electrophoresis showing the results of northern blotting hybridization where cDNA of h-l(3)mbt type 1 is hybridized to mRNAs of various human tissues.
Fig. 9 is a photograph of electrophoresis showing the results of RT-PCR carried out to normal leucocyte and cancer cell strains of human beings.
Fig. 10 shows a pGEX-2TH vector with a GST gene incorporated therein.
Fig. 11 is a photography of electrophoresis showing the results of western blotting where an antibody obtained by immunizing a rabbit with an antigen comprising the amino acid sequence set forth in SEQ ID NO: 17 of the Sequence Listing reacts with h-l(3)mbt.
Fig. 12 is a photography of electrophoresis showing the results of western blotting where an antibody obtained by immunizing a rabbit with an antigen comprising the amino acid sequence described in SEQ ID NO: 18 in the sequence listing reacts with h-l(3)mbt.
Fig. 13 is a photography of electrophoresis showing the results of western blotting where an antibody obtained by immunizing a rabbit with an antigen comprising the amino acid sequence set forth in SEQ ID NO: 1 of the Sequence Listing reacts with h-l(3)mbt.
Fig. 14 is a graph showing the results of absorbance measured by ELISA after the antibody obtained by immunizing a rabbit with an antigen comprising the amino acid sequence set forth in SEQ ID NO: 18 of the Sequence Listing reacts with h-l(3)mbt.
Fig. 15 is a fluorescence microscopic photograph showing the results of immunostaining of T98G-MG cells with the antibody obtained by immunizing a rabbit with an antigen comprising the amino acid sequence set forth in SEQ ID NO: 18 of the Sequence Listing.
Fig. 16A is a fluorescence microscopic photograph showing the results of tissue staining of nuclear-DNAs in cells with PI.
Fig. 16B is a fluorescence microscopic photograph showing the results of tissue staining of recombinant h-l(3)mbt type 1 in cells with FITC.
Fig. 16C is a fluorescence microscopic photograph showing the result of tissue staining of nuclear-DNAs in cells with PI, and tissue staining of recombinant h-l(3)mbt type 1 in cells with FITC.
Fig. 17A is a fluorescence microscopic photograph showing the results of tissue staining of nuclear-DNAs in cells with PI.
Fig. 17B is a fluorescence microscopic photograph showing the results of tissue staining of recombinant h-l(3)mbt type 2 in cells with FITC.
Fig. 17C is a fluorescence microscopic photograph showing the result of tissue staining of nuclear-DNAs in cells with PI, and tissue staining of recombinant h-l(3)mbt type 2 in cells with FITC.
Fig. 18A is a fluorescence microscopic photograph showing the results of tissue staining of nuclear-DNAs in cells with PI.
Fig. 18B is a fluorescence microscopic photograph showing the results of tissue staining of l(3)mbt in cells with FITC.
Fig. 18C is a fluorescence microscopic photograph showing the result of tissue staining of nuclear-DNAs in cells with PI, and tissue staining of l(3)mbt in cells with FITC.
Fig. 19 is a photograph of electrophoresis showing the results of northern blotting hybridization where cDNAs of m-l(3)mbt are hybridized to mRNAs of various tissues of an adult mouse.
Fig. 20 is a photograph of electrophoresis showing the results of northern blotting hybridization where cDNAs of m-l(3)mbt are hybridized to mRNAs of various tissues of a fetal mouse.
Fig. 21 is a photograph of electrophoresis showing the results of western blotting hybridization where an antibody obtained by immunizing a rabbit with an antigen comprising the amino acid sequence set forth in SEQ ID NO: 19 of the Sequence Listing reacts with m-l(3)mbt.
Fig. 22A is a microscopic photograph of a cell.
Fig. 22B is a fluorescence microscopic photograph showing the results of tissue staining of nuclear-DNAs in a cell with PI.
Fig. 22C is a fluorescence microscopic photograph showing the results of tissue staining of recombinant m-l(3)mbt in a cell with FITC.
Fig. 22D is a fluorescence microscopic photograph showing the result of tissue staining of nuclear-DNAs in a cell with PI, and tissue staining of recombinant m-l(3)mbt in a cell with FITC.

### Best Mode for Carrying Out the Invention

l(3)mbt homologues of the present invention are not limited to the proteins comprising the amino acid sequences set forth in SEQ ID NO: 1, 3, 5 or 7 in the Sequence listing but also include the protein encoded by a cDNA obtained from a cDNA library of each kind of animal by using a polynucleotide as a probe, said polynucleotide encoding said protein that comprises the amino acid sequences set forth in SEQ ID NO: 1, 3, 5 or 7 of the Sequence Listing, in particular the polynucleotide comprising the whole or a part of encoding region of a base sequences set forth in SEQ ID NO: 2, 4, 6 or 8 of the Sequence Listing.

Among h-l(3)mbt gene, m-l(3)mbt gene and r-l(3)mbt gene, the well-conservative base sequences are the parts including mbt-repeat, i.e., the part from the 865th base T to 1809th base C of the base sequence of h-l(3)mbt gene set forth in SEQ ID NO: 2 in the Sequence Listing; the part from the 1062nd base T to 2006th base C of the base sequence of m-l(3)mbt gene set forth in SEQ ID NO: 6 in the Sequence Listing; and the part from the 861st base T to 1805th base C of the base sequence of r-l(3)mbt gene set forth in SEQ ID NO: 8 in the Sequence Listing.

By using a DNA fragment comprising the above-described base sequence as a probe, a full-length 1(3)mbt gene can be obtained from a cDNA library of a target animal according to the following procedures:
1) label the probe DNA with TAKARA MEGA LABEl KIT (registered trademark, available from Takara Co. Ltd.) according to the instructions stated in the manual;
2) prepare a DNA reaction solution with the following-stated composition, incubate the solution for 30 minutes at 37°C, and then subject the solution to heating treatment for 10 minutes at 70°C to deactivate enzyme:

| | |
|---|---|
| DNA probe (2pmol/µl) | 2 µl |
| 10-fold concentrated phosphorylation buffer | 2 µl |
| [γ-32P] ATP (370MBq/ml) (available from Amersham Pharmacia Biotech Inc.) | 5 µl |
| T4 polynucleotide kinase | 1 µl |
| Total | 10 µl |

3) fill SephadexG-25 (registered trademark, available from Pharmacia Inc.) equilibrated with T50E (50mM Tris-Cl pH 8.0,1mM EDTA) in a 1.5ml POLY-PREP COLUMN (available from Bio-Rad Inc.)to make a bed volume be 1 ml, and place thereon the DNA reaction solution treated in item 2);
4) thereafter, let 200 µl of T50E to flow through the column for 4 times, thus obtaining a labeled DNA probe from the fraction eluted by the second 200 µl.
5) hybridize 150 µl of the above-obtained labeled DNA probe fraction with a cDNA probe of a plaque fixed on a nitrocellulose membrane under the following conditions: (Here, the cDNA library can be fixed onto the nitrocellulose membrane by the method disclosed in Molecular Cloning 2nd Edition(Cold Spring Harbor Laboratory Press, 1989) p.9.38-9.41)

### ① Prehybridization

6 × SSC
5 × Denhaldt's
0.05% sodium pyrophosphate
100 µg/ml denatured herring sperm DNA
0.5% SDS
Total Liquid Quantity: 50ml
Reaction temperature: 37°C
Reaction time: 1 hour

### ② Hybridization

6 × SSC
1 × Denhaldt's
0.05% sodium pyrophosphate
100 µg/ml denatured herring sperm DNA
1×10⁶cpm/ml cDNA probe
Total Liquid Quantity: 50ml
Reaction temperature: 42°C
Reaction time: 18 hours

6) after the hybridization, wash the nitrocellulose membrane once under condition ① and once under condition ② as follows:
   ① 6 × SSC, 0.1% SDS 500ml
      - temperature:: 42°C
      - time:: 20 minutes
   ② 3 × SSC, 0.1% SDS 500ml
      - temperature:: 42°C
      - time:: 20 minutes
7) expose the washed nitrocellulose membrane to a X-ray film (for example, XAR5 film available from Kodak Inc.) at -80°C for one night, and take an autoradiograph;
8) determine the location of positive plaques from the autoradiograph and collect the corresponding plaques on agar with a SM solution;
9) use the recollected plaques to form plaques again on a NZY agar culture medium by conventional means and fix the same onto a nitrocellulose membrane;
10) repeat the steps 5) to 9) for tree times to make positive plaques be single, collect this plaque, suspend it in 100µl of the SM solution, and stabilize the phage.

The gene isolated from this plaque is a 1(3)mbt homologous gene.

### 3. Large scale preparation of l(3)mbt homologous genes

1) mix 50µl of the phage of the plaque suspended in the SM solution with 20µl of *E.coli*,Y1090r, and hold the mixture still at 37°C for 15 minutes;
2) subsequently, transfer the mixed solution to 10 ml of NZY culture medium containing 100µg/ml ampicillin, and perform culture at 37°C for 6 hours;
3) carry out centrifugal separation at 8000rpm for 5 minutes, then collect the supernatant;
4) dissolve the supernatant by adding 1ml of 5M NaCl and 1.1 g of polyethylene glycol 6000;
5) hold this solution on ice for 1 hour, and then subject it to centrifugal separation at 10000rpm, 4°C for 20 minutes;
6) collect the precipitate and suspend it in 700 µl of SM solution;
7) add 500µl of chloroform and stir the suspension to dissolve the residual *E.coli*;
8) carry out centrifugal separation at 5000rpm for 10 minutes and collect the aqueous layer;
9) add each 1µl of 1mg/ml RNaseA and 5mg/ml DNaseI (both available from Sigma Inc.) to the collected aqueous layer, hold the solution at 37°C for 1 hour, and then add 600µl of 20% polyethylene glycol 6000 (0.8M NaCl) and hold the solution on ice for 30 minutes;
10) carry out centrifugal separation at 15000rpm, 4°C for 20 minutes, and collect precipitation;
11) add 500µl of SM solution, 50µl of 5M NaCl and 50µl of 0.5M EDTA to the collected precipitation, and further add 400µl of phenol and stir to dissolve the phage, thus releasing cDNA;
12) subject this solution to centrifugal separation at 15000rpm, room temperature for 5 minutes, collect the aqueous layer, and then add 1ml of ethanol to the aqueous layer, carry out centrifugal separation at 15000rpm for 20 minutes and remove the liquid layer;
13) wash the precipitate with 1ml of 70% ethanol 1ml, then dissolve the precipitate in 100 µl of TE solution (Tris-Cl pH 8.0, 10mM, 1mM EDTA) to obtain a DNA solution.

### 4. Determination of base sequences of l(3)mbt homologous genes

Full base sequences of l(3)mbt homologous genes are determined by means of dye terminator in the manner similar to Example 1. In the case where the gene obtained from each plaque has not a full length, a necessary number of overlapping genes are picked up and bonded to form a full length after digesting the overlapping portions with suitable restriction enzymes.

### 5. Determination of amino acid sequences

Amino acid sequences of l(3)mbt homologous proteins are determined from the base sequences determined in 4.

### 6. Preparation of transformants

Similar to step 9 of example 1, the above-obtained l(3)mbt homologous gene is inserted into a suitable vector (e.g., TA cloning vector) and then introduced into a host such as *E. coli*, to prepare a transformant.

Natural mutation or artificial mutation (for example, point mutation) can alter a polynucleotide without changing the principal functions of a polypeptide encoded by said polynucleotide. For l(3)mbt of the present invention, it is also possible to use this method to prepare a protein, namely a l(3)mbt mutant comprising an amino acid sequence obtained by making substitution, deletion or addition of one or more amino acids in the amino acid sequence set forth in SEQ ID NO:1, 3, 5 or 7 of the Sequence Listing.

The amino acid sequences of l(3)mbt mutants of the present invention can be determined on the basis of the base sequences of genes encoding the mutants. For example, a program on the market (e.g., MACVECTOR, registered trademark, available from Eastman Chemical Co., Ltd.) can be used.

Because of degeneracy in genetic codes, it is possible to substitute at least a part of bases in the base sequence of the polynucleotide with other kinds of bases without altering the amino acid sequence of a polypeptide produced by the polynucleotide. Therefore, the polynucleotides of this invention that encode l(3)mbt include all those containing any patterns of degeneracy.

DNA comprising the base sequence set forth in SEQ ID NO: 2 of the Sequence Listing is a naturally existing human l(3)mbt (h-l(3)mbt) type 1 gene, DNA comprising the base sequence set forth in SEQ ID NO:4 of the Sequence Listing is a naturally existing human l(3)mbt (h-l(3)mbt) type 2 gene, DNA comprising the base sequence set forth in SEQ ID NO: 6 of the Sequence Listing is a naturally existing mouse l(3)mbt (m-l(3)mbt) gene, and DNA comprising the base sequence set forth in SEQ ID NO: 8 of the Sequence Listing is a naturally existing rat l(3)mbt (r-l(3)mbt) gene.

The present invention includes antisense polynucleotides having base sequences of antisense strands of polynucleotides encoding the foregoing l(3)mbt, and derivatives thereof. The antisense polynucleotide can be hybridized to the polynucleotide encoding l(3)mbt, and it can inhibit the biosynthesis of a polypeptide encoded by the polynucleotide when the hybridized polynucleotide is a polynucleotide of the encoding region.

The antisense polynucleotide for inhibiting the biosynthesis of polypeptide is preferably composed of 15 or more bases. On the other hand, if the length is too long it is unsuitable for incorporating the full-length antisense polynucleotide into a cell. The antisense polynucleotide used for inhibiting the biosynthesis of l(3)mbt by incorporating it into a cell comprises 12 to 30, preferably 15 to 25, more preferably 18 to 22 bases.

The antisense polynucleotides or a part thereof according to this invention embrace all of those in which a plurality of nucleotides comprising bases, phosphoric acids, and sugars are bonded, including those not present in nature. Typical examples of antisense polynucleotides include antisens DNA and antisense RNA.

Various kinds of antisense polynucleotide derivatives having high binding strength with target DNA and mRNA, high tissue selectivity, cell permeability, nuclease resistance and intracellular stability can be obtained by employing publicly known technology to the antisense polynucleotides of the present invention.

In the viewpoint of the ease of hybridization, it is generally considered advantageous that a polynucleotide or a derivative thereof is designed to have a base sequence complementary to the base sequence of a region which forms a stem loop. The antisense polynucleotide and the derivative thereof according to this invention can form a stem loop if necessary.

It is also expected that the antisense polynucleotides having sequences complementary to sequences existing in the vicinity of the translation start codon, or at a ribosomal binding site, the capping site or a splicing site are generally provided with a great inhibitory effect on expression. Thus, a great expression inhibitory effect is expected in the polynucleotides or derivatives thereof according to this invention which contain sequences complementary to sequences existing in the vicinity of the translation start codon, or at a ribosomal binding site, the capping site, or a splicing site of a gene encoding l(3)mbt or mRNA.

At present, it is generally known that preferable derivatives are those which have an enhanced performance in at least one of nuclease resistance, tissue selectivity, cell permeability, and binding strength and that especially preferable derivatives are polynucleotide derivatives which have phosphorothioate bonds as their skeleton structures. The polynucleotides or derivatives thereof according to this invention also embrace derivatives having these functions and structures.

If the antisense polynucleotides of the present invention are natural type, they can be obtained by synthesis using a chemical synthesizer or by the PCR method that employs the gene encoding l(3)mbt as a template. Also, among the derivatives, there are ones that can be synthesized using a chemical synthesizer, such as the methylphosphonate type and the phosphorothioate type. In this case, the synthesizing operation may be conducted according to the manual attached to the chemical synthesizer and the synthesized products as obtained can be purified by HPLC method using reverse phase chromatography or other methods to produce the desired antisense polynucleotides or derivatives thereof.

The polynucleotide encoding l(3)mbt of the present invention, the antisense polynucleotide thereof, or a part thereof (polynucleotide having a base sequence comprising 12 or more consecutive bases) can be used as a probe for screening cDNA libraries or the like for the l(3)mbt genes. Here, those with GC contents of from 30 to 70% are preferably usable. Further, the polynucleotide having a base sequence comprising 15 or more consecutive bases is particularly preferably. The polynucleotides that are used as probes may be the derivatives thereof. The sequence comprising a greater number of bases than that described above is recognized as a specific sequence. With respect to cDNA libraries that are used for screening with the aid of said probes, those prepared from mRNAs can preferably be used. A group of cDNAs selected by random sampling from these cDNA libraries may be employed as samples for screening. Commercially available cDNAs are also usable.

A DNA having 12 or more consecutive bases of the base sequence set forth in SEQ ID NO:2,4 or 6 of the Sequence Listing or a polynucleotide that hybridizes to said DNA (antisense polynucleotide) can be used as the probe to screen the cDNA libraries for l(3)mbt genes.

The tissues with the expression of mRNAs derived from l(3)mbt genes can be discovered by means of northern blotting hybridization of mRNA derived from each tissue by using the polynucleatide, the antisense polynucleotide thereof or a part thereof as the probe.

It is possible to obtain a transformant by introducing the plasmid into a suitable host such as *E. coli*, as a publicly known art. l(3)mbt or l(3)mbt variants can be produced by culturing a transformant to which a l(3)mbt gene has been introduced, amplifying the gene, and expressing protein. Subsequently, a product thus cultured is recovered, and through manipulations such as concentration, solubilization, dialysis, and various chromatographic techniques as occasion calls, the l(3)mbt and l(3)mbt variants according to this invention can be purified.

A variety of references are available for the cultivation of transformants, and the preparation of l(3)mbt or l(3)mbt variants on the basis of the base sequences described in the present invention can be carried out by using a publicly known method. In this case, any of bacteria such as *E. coli*, yeast, and animal cells is usable as a host. Particularly, animal cells are preferable. For the introduction of genes into cells, the liposome method, the electroporation method, and the like can be used. Particularly, it is preferable to use the intranuclear microinjection.

For purifying l(3)mbt or l(3)mbt variants from the resulting cultured products, there are methods, such as immunoprecipitation, salting out, ultrafiltration, isoelectric precipitation, gel filtration, electrophoresis, ion-exchange chromatography, a variety of affinity chromatography such as hydrophobic chromatography and antibody chromatography, chromatofocusing, adsorption chromatography, and reverse phase chromatography, and it may be carried out by selecting any of them as appropriate.

Also, in the production stage, l(3)mbt or l(3)mbt variants to be produced may be prepared by transformants as fusion peptides with other polypeptides. In this case, the purification step requires a manipulation that slices out the l(3)mbt or l(3)mbt variants by treatment with a chemical substance such as bromocyanogen or with an enzyme such as protease.

In respect to the antigenicity of l(3)mbt, it is clarified by Example 6, 7, 8 or 9 of the present invention clarifies that an antibody can be easily obtained by immunizing an animal with the l(3)mbt obtained by the method described above or an oligopeptide comprising an amino acid sequence specific to the l(3)mbt wherein the animal is other than human being and the l(3)mbt is not derived from the animal. Therefore, an antibody recognizing l(3)mbt of the present invention (referred to as l(3)mbt antibody hereinafter) is the antibodies obtained by immunizing the animals with the l(3)mbt wherein the animal is other than human being and the l(3)mbt is not derived from the animal. Such antibody includes those whose ability of recognizing l(3)mbt of the present invention is confirmed by western blotting method, ELISA method, immunostaining method (for example, measurement by means of FACS) or the like.

Also, the method using a part of protein, which may be bound to another carrier protein such as bovine serum albumin, as the immunogen is often employed. The part of protein may be synthesized by using, for example, a peptide synthesizer. This part of protein is preferably composed of 8 or more amino acid residues.

It is well known that for the substance with clarified antigenicity, if a polyclonal antibody can be obtained by immunization, the monoclonal antibody can be produced by hybridoma using the lymphocyte of the immunized animal. ("Antibodies, A Laboratory Manual," (Cold Spring Harbor Laboratory Press, 1988) Chapter 6). Therefore, l(3)mbt antibody of the present invention also embraces the monoclonal antibody.

In the present invention, the antibody also embraces active fragments. The active fragment is a fragment of the antibody having antigen-antibody reactivity, such as F(ab')₂, Fab', Fab and Fv.

For example, F(ab')₂ is obtained if the antibody is digested with pepsin, and Fab is obtained if digested with papain. Fab' is obtained if F(ab')₂ is reduced by an reagent such as 2-mercaptoethanol and alkylated by monoiodoacetic acid. Fv is a univalent antibody active fragment wherein a heavy chain variable region and a light chain valiable region are bonded to each other via a linker.

Chimera antibody is obtained by keeping the above-stated active fragment while substituting fragments of other animals for the other parts.

The antibody of the present invention is usable for the clarification of functions of l(3)mbt expressions or the like and the clarification of generation mechanisms of malignant tumors.

Detection of l(3)mbt may be performed by the methods using antibodies or the methods employing enzyme reactions.

Specific examples of the methods using antibodies include (1) the methods using labeled l(3)mbt antibody to detect l(3)mbt; and(2) the method using l(3)mbt antibody and labeled secondary antibody thereof to detect l(3)mbt. The markers used here include, for example, radio isotopes (RI), enzymes, avidin or biotin, or fluorescent substances (FITC, rhodamine, etc.).

Examples of the methods employing enzyme reaction include ELISA method, immunoagglutination, western blotting method, identification of immunoreactive molecules using flow cytometry, or the methods similar thereto.

This invention will further be detailed with the illustration of examples below; however, the invention is not to be limited to these examples.

### (Example 1)

### Isolation of h-l(3)mbt Genes and Determination of Base Sequences

### 1. Searching EST Database

When searching for the sequences having homology with *drosophila* l(3)mbt genes from the sequences (EST) registered in EST database (database wherein sequences of mRNA fragments are registered,http://www.ncbi.nlm.nih.gov./i html) on internet was conducted, a registered sequence, H23073 was hit.

### 2. Synthesis of Primers

In order to acquire DNA fragments each comprising a base sequence which is a part of the base sequence of the foregoing H23073 from human cDNA library, the following primers were synthesized.

P1 primer 5'-CCATCAAAGT GCAGGCGTAG G-3' (base sequence set forth in SEQ. ID. NO: 20 of the Sequence Listing) and P2 primer 5'-TAGCCACATA CCTCAGCCAC G-3' (base sequence set forth in SEQ. ID. NO: 21 of the Sequence Listing) were designed and synthesized with a DNA synthesizer (ABI Model 392) as a 5' primer and a 3' primer, respectively. Each of the synthesized primers was dissolved in distilled water to give a concentration of 20 pmol/µl. These solutions were used as PCR primers to perform PCR operations described as follows:

### 3. PCR (1)

PCR operation using a human fetal brain cDNA library(available from Clontech Inc.) as the cDNA library was conducted under the following conditions.

PCR only towards the 5'-end was conducted by only employing P2 primer as the primer. The PCR operation included the steps of placing a solution having the following-stated composition into a test tube, overlaying mineral oil (15µl) on the solution, and allowing it to stand at 94 ° C for 5 min.

| | |
|---|---|
| cDNA library (1µg/µl) | 2 µl |
| dNTP mixed solution (respective NTPs were dissolved in distilled water at concentrations of 20 pmol/µl) | 1 µl |
| P2 Primer (20 pmol/µl) | 1 µl |
| 10-fold concentrated PCR buffer (400 mM Tris-HCl (pH 8.3), 1M KCl, 100 mM MgCl₂, 100 mM DTT) | 1.5 µl |
| distilled water | 9 µl |
| Taq polymerase (5 units/µl) | 0.5 µl |
| Total | 15 µl |

Subsequently, the following cycle was repeated 50 times for reaction: at 60 ° C for 30 sec, followed by at 72 ° C for 1.5 min, and followed by at 94 ° C for 30 sec.

Then the reaction was allowed to proceed at 55°C for 2 min, finally at 72 ° C for 10 min to carry out the elongation reaction of the fragment, thus completing the PCR operation.

Next, this PCR product was used as a template to perform the second PCR as follows: A solution having the following composition was placed into a test tube. Mineral oil (20 µl) was overlaid on the solution and it was allowed to stand at 94 ° C for 5 min:

| | |
|---|---|
| The aforementioned PCR product (1µg/1µl) | 5 µl |
| dNTP mixed solution | 2 µl |
| P1 Primer(20 pmol/µl) | 1 µl |
| 10-fold concentrated PCR buffer (400 mM Tris-HCl (pH 8.3), 1 M KCl, 100 mM MgCl₂, 100 mM DTT)) | 2 µl |
| distilled water | 9.5 µl |
| Tag polymerase (5 units/µl) | 0.5 µl |
| Total | 20 µl |

Subsequently, the following cycle was repeated 40 times for reaction: at 60 ° C for 30 sec, followed by at 72 ° C for 1 min, and followed by at 94 ° C for 30 sec.

Then, reaction was allowed to proceed at 55 ° C for 2 min and finally at 72° C for 10 min to carry out the elongation reaction of the fragment, thus completing the PCR operation.

### 4. Determination of DNA Base Sequence (1)

The DNA fragment (referred to as fragment A hereinafter) obtained from the above-described 5'-end elongation reaction was subjected to the mini gel electrophoresis (0.75% agarose gel) to cut a band of the fragment A out of the gel. The fragment A was recovered by means of GENE CLEAN (available from BIO 101 Inc.), and the band was ascertained by means of the mini gel electrophoresis.

1 µl of the fragment A was taken up and diluted in 99 µl of TE. The absorbance (A260) of the diluted solution was measured at 260 nm and the DNA concentration was computed (A260, 1.0= 50 µg/ml). The fragment A was further diluted with TE so that its DNA concentration reached 1 µg/µl.

For this diluted solution, DNA sequencing was conducted by dye-terminator method using T7 primer and an auto-sequencer(ABI Model 373A), so as to determine the base sequence of the fragment A. This base sequence is set forth in SEQ ID NO: 9 of the Sequence Listing.

### 5. PCR(2)

The elongation reaction toward 3'-end was conductor in the same manner of the elongation reaction towards 5'-end as described in the foregoing item 3. P3 primer 5'-AAGCTGTTTG ACCGCATGAA C-3'(the base sequence set forth in SEQ ID NO:22 of the Sequence Listing) was designed as the 5'-primer, and P4 primer 5'-AAGCACCTGT TTGTGAGCCA G-3'(the base sequence set forth in SEQ ID NO: 23 of the Sequence Listing) was designed as the 3'-primer. The primers were synthesized with a DNA synthesizer (ABI model 392). The synthesized DNA primers were dissolved in distilled water to give a concentration of 20 pmol/µl.

### 6. Determination of Base Sequence (2)

The base sequence of the DNA fragment (referred to as fragment B hereinafter) obtained from the above-described 3'-end elongation reaction was determined by the same means recited in the foregoing 4. This base sequence is set forth in SEQ ID NO: 10 of the Sequence Listing.

### 7. Acquisition of h-l(3)mbt Genes

A sense primer, P5 primer 5'-ATGAGGCGAA GAGAGGGCCA TG-3'(the base sequence set forth in SEQ ID NO: 24 of the Sequence Listing) corresponding to a part of the fragment A, and an antisense primer, P6 primer 5'-GTTAGGATCC TTGCAGTAAA TCAC-3' (the base sequence set forth in SEQ ID NO: 25 of the Sequence Listing) corresponding to a part of the fragment B were synthesized with a DNA synthesizer (ABI Model 392) as the 5'-primer and the 3'-primer, respectively.

PCR reaction was conducted by using these primers and using a human fetal brain cDNA library (available from Clontech Inc.) as the template. A solution having the following composition was placed into a test tube, mineral oil (20 µl) was overlaid on the solution, and it is allowed to stand at 94 ° C for 5 min:

| | |
|---|---|
| human fetal brain cDNA library (1µg/µl) | 5 µl |
| dNTP mixed solution (respective NTPs were dissolved in distilled water at concentrations of 20 pmol/µl) | 1 µl |
| P5 Sense Primer (20 pmol/µl) | 1 µl |
| P6 Antisense Primer (20 pmol/µl) | 1 µl |
| 10-fold concentrated PCR buffer (400 mM Tris-HCl (pH 8.3), 1M KCl, 100 mM MgCl₂, 100 mM DTT) | 2 µl |
| Taq polymerase (5 units/µl) | 0.5 µl |
| distilled water | 9.5 µl |
| Total | 20 µl |

Subsequently, the following cycle was repeated 40 times for reaction: at 60 ° C for 30 sec, followed by at 72 ° C for 1 min, and followed by at 94 ° C for 30 sec. Then the reaction was allowed to proceed at 55°C for 2 min, finally at 72 ° C for 10 min, thus completing the PCR operation.

After the reaction, the PCR product was subjected to the mini gel electrophoresis (0.75% agarose gel). As a result, a band of about 2.4kbp was observed.

Thus, the h-l(3)mbt gene was isolated.

### 8. Determination of Base Sequence (3)

A band of the h-l(3)mbt gene obtained as described above was cut out of the gel. The h-l(3)mbt gene was recovered by means of GENE CLEAN (available from BIO 101 Inc.), and the band was ascertained by means of the mini gel electrophoresis.

1 µl of the h-l(3)mbt gene was taken up and diluted in 99 µl of TE. The absorbance (A260) of the diluted solution was measured at 260 nm and the DNA concentration was computed (A260, 1.0= 50 µg/ml). The h-l(3)mbt gene solution was further diluted with TE so that its DNA concentration reached 1 µg/µl.

For this diluted solution, DNA sequencing was conducted by dye-terminator method using T7 primer and the auto-sequencer(ABI Model 373A), so as to determine the base sequence of the h-l(3)mbt gene. As a result, it was found that an alternative splice form existed in h-l(3)mbt genes. The short gene is referred to as type 1 and the long gene is referred to as type 2. The base sequence of h-l(3)mbt type 1 gene is set forth in SEQ ID NO: 2 of the Sequence Listing and the base sequence of h- h-l(3)mbt type 2 gene is set forth in SEQ ID NO: 4 of the Sequence Listing. Type 2 is a base sequence wherein 118 bases are inserted in vicinity of C-terminus of type 1, specifically between 2373th and 2374th bases of the base sequence set forth in SEQ ID NO: 2 of the Sequence Listing.

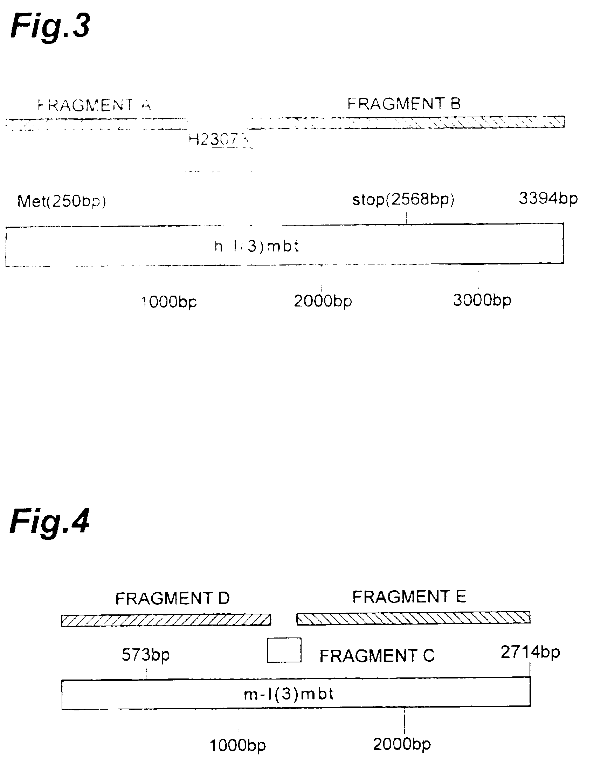

The location relationship between the fragments A and B obtained in the foregoing process are shown in Fig. 3.

### 9. Determination of Amino Acid Sequence

The amino acid sequences of the h-l(3)mbt were determined based on the above-described base sequences. The amino acid sequence of the h-l(3)mbt type 1 is set forth in SEQ ID NO: 1 of the Sequence Listing and the amino acid sequence of the h-l(3)mbt type 2 is set forth in SEQ ID NO: 3 of the Sequence Listing. The type 1 and the type 2 differ from each other from the 709th amino acid.

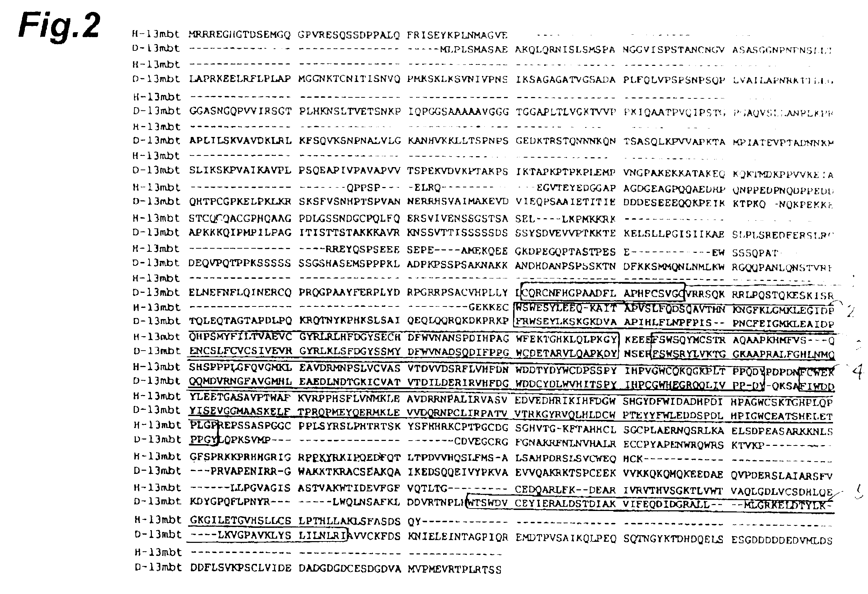

The amino acid sequences of d-l(3)mbt and h-l(3)mbt are shown in Fig. 2. In this figure, the portions of zinc finger domains, mbt repeat and protein rich domains are enclosed in boxes. It was found that the rate of concordance of d-l(3)mbt and h-l(3)mbt at mbt repeat was 43.7% and both d-l(3)mbt and h-l(3)mbt were well conserved in mbt repeat. Therefore, mbt-repeat is considered to play a significant role in making the protein exhibit its function.

### 10. Preparation of Transformants

A full-length h-l(3)mbt type 1 gene comprising the above-mentioned base sequence was inserted into a TA cloning vector and transformation was carried out by introducing said vector to *E. coli*(DH5α). This transformant was named TA-h-l(3)mbt and deposited on July 9, 1997 in the National Institute of Bioscience and Human-Technology, Agency of Industrial Science and Technology, Ministry of International Trade and Industry locating at 1-1-3 Higashi, Tsukuba, Ibaragi, Japan (Accession No. FERM P-16317)and it was transferred on July 3, 1998 to International Deposition (Accession No. FERM BP-6418).

### (Example 2)

### Isolation of m-l(3)mbt Gene, Determination of The Base Sequence of The Gene, and Determination of Amino Acid Sequence Encoded by Said Gene

### 1. Acquisition of DNA fragments

A mouse neonatal brain cDNA library (λ - ZAPII) was amplified by using P7 primer 5'-TGAACTCCTC CTCCTTGTAA CCT-3' (the base sequence set forth in SEQ ID NO:26 of the Sequence Listing) and P8 primer 5'-GTCCATGTAC TTCATCCTCA C-3' (the base sequence set forth in SEQ ID NO:27 of the Sequence Listing). DNA sequencing was conducted by dye-terminator method using T7 primer and the auto-sequencer(ABI Model 373A), so as to determine the base sequence of the above-obtained DNA. As a result, a DNA fragment composed of a base sequence having homology to a part of the base sequence of the foregoing H23073 was successfully acquired. (This DNA fragment is referred to as fragment C hereinafter and the base sequence of the fragment C is set forth in SEQ ID NO: 11 of the Sequence Listing.)

### 2. Synthesis of Primers

M13 reverse primer 5'-CAGGAAACAG CTATGAC-3' (the base sequence in λ -ZAPII and set forth in SEQ ID NO:28 of the Sequence Listing)and P7 primer were designed as primers and synthesized with the DNA synthesizer (ABI Model 392). The synthesized primers were dissolved in distilled water to give a concentration of 20 pmol/µl. The solution was used as PCR primers to perform the PCR operation described as follows.

### 3. PCR (1)

PCR operation using a mouse neonatal brain cDNA library was conducted under the following conditions.

The PCR operation included the steps of placing a solution having the following composition into a test tube, overlaying mineral oil (15 µl) on the solution, and allowing it to stand at 94 ° C for 5 min.

| | |
|---|---|
| mouse neonatal brain cDNA library (1µg/µl) | 2 µl |
| dNTP mixed solution (respective NTPS were dissolved in distilled water at concentrations of 20 pmol/LLl) | 1 µl |
| M13 Reverse Primer (20 pmol/µl) | 1 µl |
| P7 Primer (20 mol/µl) | 1 µl |
| 10-fold concentrated PCR buffer (400 mM Tris-HCl (pH 8.3), 1M KCl, 100 mM MgCl₂, 100 mM DTT) | 1.5 µl |
| Taq polymerase (5 units/µl) | 0.5 µl |
| distilled water | 8 µl |
| Total | 15 µl |

Subsequently, the following cycle was repeated 30 times for reaction: at 60 ° C for 30 sec, followed by at 72 ° C for 1.5 min, and followed by at 94 ° C for 30 sec.

Then the reaction was allowed to proceed at 55°C for 2 min, finally at 72 ° C for 10 min to carry out the elongation reaction of the fragment, thus completing the PCR operation.

Next, this PCR product was used as a template to perform the second PCR using T3 primer 5'-AATTAACCCT CACTAAAGGG-3' (the base sequence in λ -ZAPII and set forth in SEQ ID NO:29 of the Sequence Listing)and P1 primer. The second PCR operation included the steps of placing a solution having the following composition into a test tube, overlaying mineral oil (20 µl) on the solution and allowing it to stand at 94 ° C for 5 min:

| | |
|---|---|
| The aforementioned PCR product (1µg/1µl) | 5 µl |
| dNTP mixed solution | 2 µl |
| T3 Primer(20 pmol/µl) | 1 µl |
| P1 Primer(20 pmol/µl) | 1 µl |
| 10-fold concentrated PCR buffer (400 mM Tris-HCl (pH 8.3), 1 M KCl, 100 mM MgCl₂, 100 mM DTT)) | 2 µl |
| Taq polymerase (5 units/µl) | 0.5 µl |
| distilled water | 8.5 µl |
| Total | 20 µl |

Subsequently, the following cycle was repeated 35 times for reaction: at 60 ° C for 30 sec, followed by at 72 ° C for 1 min, and followed by at 94 ° C for 30 sec.

Then, reaction was allowed to proceed at 55 ° C for 2 min and finally at 72° C for 10 min to carry out the elongation reaction of the fragment, thus completing the PCR operation.

### 4. Determination of Base Sequence (1)

The DNA fragment (referred to as fragment D hereinafter) obtained from the above-described 5'-end elongation reaction was subjected to the mini gel electrophoresis (0.75% agarose gel) to cut a band of the fragment A out of the gel. The fragment D was recovered by means of GENE CLEAN (available from BIO 101 Inc.), and the band was ascertained by means of the mini gel electrophoresis.

1 µl of the fragment D was taken up and diluted in 99 µl of TE. The absorbance (A260) of the diluted solution was measured at 260 nm and the DNA concentration was computed (A260, 1.0= 50 µg/ml). The fragment D solution was further diluted with TE buffer so that its DNA concentration reached 1 µl/µl.

For this diluted solution, DNA sequencing was conducted by dye-terminator method using T3, P1 primer and the auto-sequencer(ABI Model 373A), so as to determine the base sequence of the fragment D. This base sequence is set forth in SEQ ID NO: 12 of the Sequence Listing.

### 5. PCR(2)

The elongation reaction toward 3'-end was conductor in the same manner of the elongation reaction towards 5'-end as described in the foregoing item 3. In the first PCR operation, M13 primer and P8 primer were designed, and in the second PCR, T3 primer and P6 primer are designed, and the primers were synthesized with the DNA synthesizer (ABI model 392). The synthesized DNA primers were dissolved in distilled water to give a concentration of 20 pmol/µl.

### 6. Determination of Base Sequence (2)

The base sequence of the DNA fragment (referred to as fragment E hereinafter) obtained from the above-described 3'-end elongation reaction was determined by the same means recited in the foregoing item 4. This base sequence is set forth in SEQ ID NO: 13 of the Sequence Listing.

### 7. Acquisition of m-l(3)mbt genes

A sense primer, P9 primer 5'-GTTGAAATGC TGGCGTAGTC-3' (the base sequence set forth in SEQ ID NO: 30 of the Sequence Listing) corresponding to a part of the fragment D, and a antisense primer, P10 primer 5' -CCCAGTCAGT CACTTAAAGA CATC-3'(the base sequence set forth in SEQ ID NO: 31 of the Sequence Listing) corresponding to a part of the fragment E were synthesized with the DNA synthesizer (ABI Model 392) as the 5'-primer and the 3'-primer, respectively.

PCR reaction was conducted by using these primers and a mouse fetal brain cDNA library (available from Clontech Inc.) as the template. A solution having the following composition was placed into a test tube, mineral oil (20 µl) was overlaid on the solution, and it was allowed to stand at 94 ° C for 5 min:

| | |
|---|---|
| mouse neonatal brain cDNA library (1µg/µl) | 5 µl |
| dNTP mixed solution (respective NTPs were dissolved in distilled water at concentrations of 20 pmol/µl) | 1 µl |
| P9 Sense Primer (20 pmol/µl) | 1 µl |
| P10 Antisense Primer (20 pmol/µl) | 1 µl |
| 10-fold concentrated PCR buffer (400 mM Tris-HCl (pH 8.3), 1M KCl, 100 mM MgCl₂, 100 mM DTT) | 2 µl |
| Taq polymerase (5 units/µl) | 0.5 µl |
| distilled water | 9.5 µl |
| Total | 20 µl |

Subsequently, the following cycle was repeated 40 times for reaction: at 60 ° C for 30 sec, followed by at 72° C for 1 min, and followed by at 94 ° C for 30 sec. Then the reaction was allowed to proceed at 55°C for 2 min, finally at 72 ° C for 10 min, thus completing the PCR operation.

After the reaction, the PCR product was subjected to the mini gel electrophoresis (0.75% agarose gel). As a result, a band of about 2.6kbp was observed.

Thus, the m-l(3)mbt gene was isolated.

### 8. Determination of Base Sequence (3)

A band of m-l(3)mbt gene obtained as described above was cut out of the gel. The m-l(3)mbt gene was recovered by means of GENE CLEAN (available from BIO 101 Inc.), and the band was ascertained by means of the mini gel electrophoresis.

1 µl of the m-l(3)mbt gene was taken up and diluted in 99 µl of TE. The absorbance (A260) of the diluted solution was measured at 260 nm and the DNA concentration was computed (A260, 1.0= 50 µg/ml). The m-l(3)mbt gene solution was further diluted with TE buffer so that its DNA concentration reached 1 µg/µl.

For this diluted solution, DNA sequencing was conducted by the dye-terminator method using T7 primer and the auto-sequencer(ABI Model 373A), so as to determine the base sequence of the m-l(3)mbt gene. The base sequence is set forth in SEQ ID NO: 6 of the Sequence Listing.

The location relationship of the fragments C, D and F obtained in the foregoing processes are shown in Fig. 4.

### 9. Determination of Amino Acid Sequence

The amino acid sequence of the m-l(3)mbt was determined based on the above-described base sequence. The amino acid sequence of the m-l(3)mbt is set forth in SEQ ID NO: 5 of the Sequence Listing.

The homology between the h-l(3)mbt genes and the m-l(3)mbt gene is 87%, and the homology between h-l(3)mbt protein and m-l(3)mbt protein is 70%, so that they are very well conserved between species.

### 10. Preparation of Transformants

A full-length m-l(3)mbt gene comprising the above-mentioned base sequence was incorporated into a TA cloning vector and the transformant was carried out by introducing said vector to *E. coli*(DH5α).

### (Example 3)

### Isolation of rat-l(3)mbt Gene (r-l(3)mbt gene), Determination of The Base Sequence of The Gene, and Determination of The Amino Acid Sequence Encoded by Said Gene

The isolation of the r-l(3)mbt gene and the determination of the base sequence of said gene were conducted in the manner similar to that of Example 2 regarding the case of mouse. The procedures will be briefly described hereinafter.

### 1. Acquisition of DNA fragments

The cDNA library used in this example was a rat neonatal brain cDNA library prepared by the present inventors with a kit provided by GIBCOBRL Inc. A fragment F having high homology with H23073 was acquired by means of PCR using the above-mentioned P8 primer and P11 primer 5'-ATCCAGAAAT CATAGCCATG ACT-3' (the base sequence set forth in SEQ ID NQ:32 of the Sequence Listing). The base sequence of the fragment F is set forth in SEQ ID NO: 14 of the Sequence Listing.

### 2.PCR(1)

The cloning towards 5'-end was conducted by only once PCR using the P11 primer and P12 primer 5'-GTTGAAATGC TGGCGTAGTC C-3'(the base sequence set forth in SEQ ID NO: 33 of the Sequence Listing). The cycle of PCR was conducted for 40 times. As a consequence, a fragment G was obtaind. The base sequence of the fragment G was set forth in SEQ ID NO: 15 of the Sequence Listing.

### 3. PCR(2)

The cloning toward 3'-end was conducted by a first PCR, wherein only P8 primer was used and the cycle was conducted for 50 times, and a second PCR, wherein the above-mentioned P5 primer and P13 primer 5'-GGCCACGCGT CGACTAGTAC-3' (an adapter primer of 3'RACE KIT (available from Boehringer Mannheim Inc.), the base sequence set forth in SEQ ID NO: 34 of the Sequence Listing) were used and the cycle was conducted for 40 times. As a consequence, a fragment H was obtained. The sequence of the fragment H was set forth in SEQ ID NO: 16 of the Sequence Listing.

### 4. Determination of base sequence

The base sequence of a full-length r-l(3)mbt gene was determined based on the above-obtained fragments F, G and H. This base sequence is set forth in SEQ ID NO: 8 of the Sequence Listing.

### 5. Determination of amino acid sequence

The amino acid sequence of r-l(3)mbt was determined from the foregoing base sequence. This amino acid sequence was set forth in SEQ ID NO: 7 of the Sequence Listing.

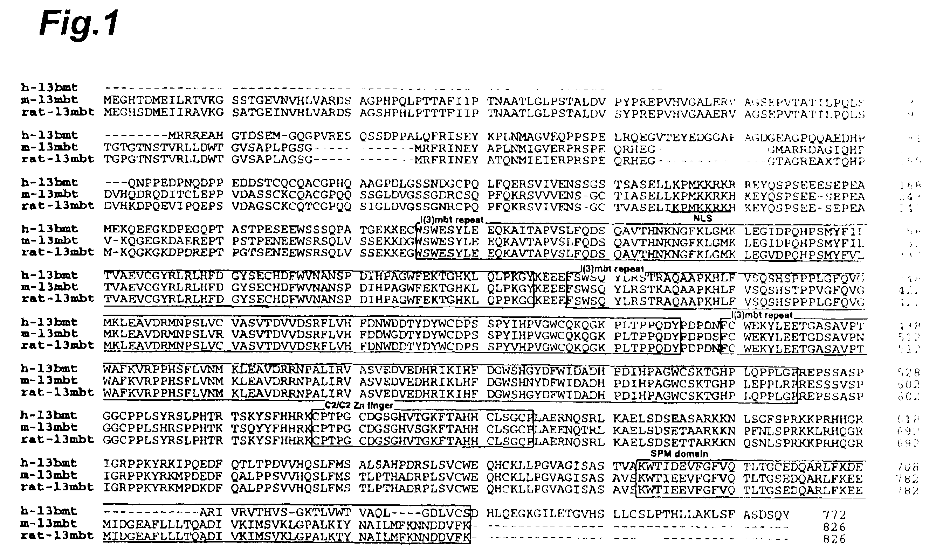

The homology between m-l(3)mbt and the r-l(3)mbt is 90% or more, so that they are very well conserved between species. The amino acid sequences of h-l(3)mbt, m-l(3)mbt and r-l(3)mbt are shown in Fig. 1. In this figure, the portions of mbt repeat are enclosed in boxes. It was found that the rate of concordance of the amino acid in mbt repeat was 95.0% among h-l(3)mbt, m-l(3)mbt and r-l(3)mbt, and they were very well conserved. As a consequence, mbt-repeat is considered to play a significant role when the protein exhibits its function.

### (Example 4)

### Expression of h-l(3)mbt in a Variety of Human Tissues

The labeled full-length h-l(3)mbt type 1 gene was hybridized to each membrane(HUMAN TISSUE NORTHERN BLOT I, II and HUMAN CELL LINE MULTIPLE TISSUE NORTHERN BLOT (available from Clontech Inc.)) on which 2 µg of poly(A)+RNA (mRNA) from a variety of human tissues and poly(A)+RNA (mRNA) from a variety of human cells were blotted, according to the description in Molecular Cloning: A Laboratory Manual 2nd Edition, p 7.39-7.52, thus the analysis by means of northern blotting hybridization was conducted.

Labeling of h-l(3)mbt gene was performed by the following procedures:
1)label the gene with a TAKARA MEGA LABELING KIT (registered trademark, available from Takara Co. Ltd.) according to the procedures described in the manual; and
2)then, prepare a DNA reaction liquid having the following composition, incubate this liquid at 37°C for 30 minutes, and then subject the liquid to heat treatment for 70°C for 10 minutes, thus deactivating the enzyme:

| | |
|---|---|
| DNA probe (2pmol/µl) | 2 µl |
| 10-fold concentrated phosphorylation buffer | 2 µl |
| [γ-³²P]ATP(370MBq/ml) (available from available from Amersham Pharmacia Biotech Inc.) | 5 µl |
| T4 polynucleotide kinase | 1 µl |
| Total | 10 µl |

3) fill SEPHADEX G-25 (registered trademark, available from Pharmacia Inc.) equilibrated by T50E (50mM Tris-Cl pH 8.0,1mM EDTA) in 1.5ml POLY-PREP COLUMN (available from Bio-Rad Inc.) to obtain a bed volume of 1 ml, and place on this column the DNA reaction liquid subjected to the heat treatment in the aforementioned step 2);
4) thereafter, flow 200 µl of T50E through the column for 4 times to obtain a labeled DNA probe from the fraction eluted by the second 200µl.

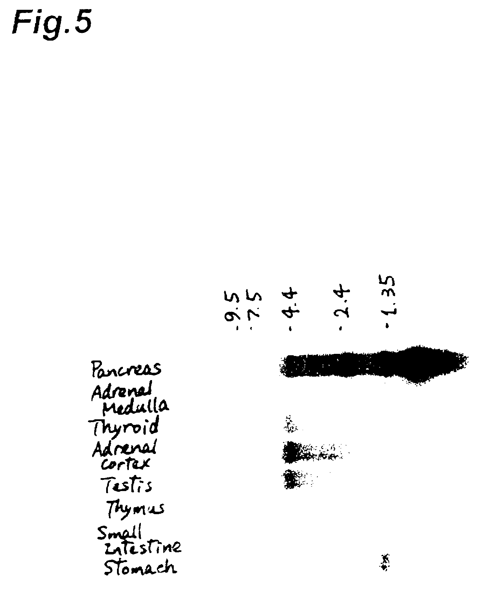

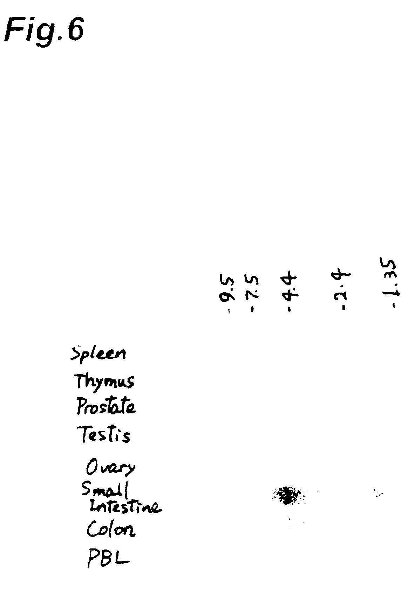

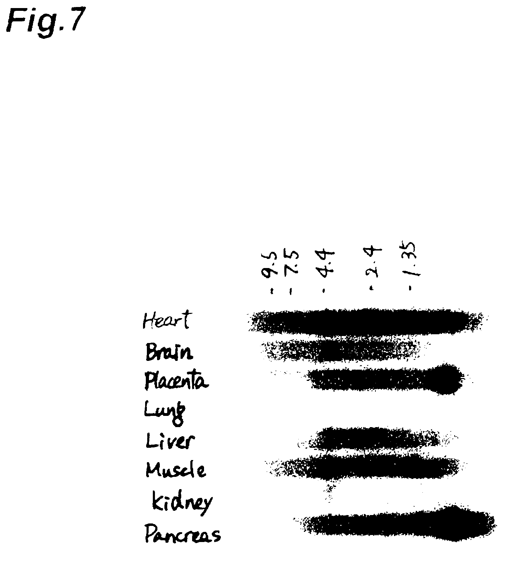

Photographs showing the results in respect to a variety of tissues are shown in Figs. 5 to 7. The expression of h-l(3)mbt (a band of 5.5kb) was found in all tissues from these photographs.

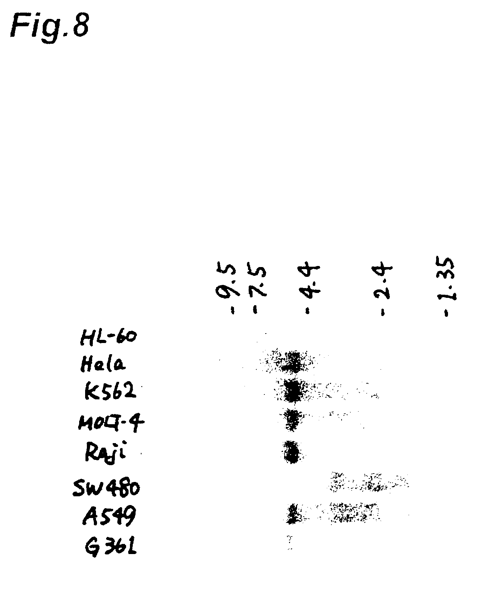

Fig. 8 is a photograph showing results in respect to a variety of cells. From this photograph, the expression was recognized in all the cells except SW480.

### (Example 5)

### Expression of Alternative Splice Form of h-l(3)mbt Gene

The expression ratio of the h-l(3)mbt type 1 gene to the h-l(3)mbt type 2 gene was analyzed by means of RT-PCR.

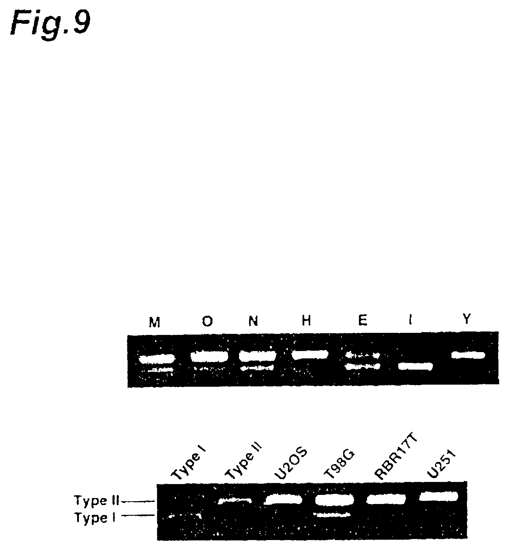

mRNAs of normal human leukocyte (from seven volunteers) and cancer cell strains (U20S, T98G, RBR17T and U251)were subjected to RT-PCR using the above-described P5 primer and P6 primer, and the PCR products were electrophoresed. The results are shown in Fig. 9. The upper portion of Fig. 9 shows the results in respect of human beings, and the alphabetical letters of each lane are the identification codes of the volunteers. The upper bands represent the bands of type 2 and the lower ones represent the bands of type 1. Fig. 9 shows the dominant expressions of type 2 in five people's normal leukocyte and cancer cell strains. Further, the dominant expression of type 1 was found in one person's normal leukocyte, and the somewhat dominant expression of type 1 was found in the other one person's normal leukocyte.

### (Example 6)

### Production of Recombinant h-l(3)mbt Protein

Full-length h-l(3)mbt type 1 genes were incorporated into a pCGN vector and a pBJ-Myc vector. Each of the vectors was introduced into COS cell to produce transformant. The following operation were the same to either kind of the transformants.

1x10⁶ transformants were added into a cell lysing solution (100 µl)comprising 50 mM Tris-HCl, 150 mM NaCl, 1% Triton X-100, 50 mM iodoacetamide, 2 mM MgCl₂, 2 mM CaCl₂, 0.1% NaN₃, 10 µg/ml soybean trypsin inhibitor, 1 µg/ml aprotinin, 1 mM PMSF (phenyl methyl sulfonyl fluoride) and 1 µg/ml leupeptin, and dissolved by stirring. After standing on ice for 60 min, the solution was centrifuged at 15,000 rpm for 10 min and the supernatant was recovered.

To the recovered supernatant was added 50 ml of the CNBr-activated Sepharose beads (available from Amersham Pharmacia Biotech Inc.; antibody concentration of 1 mg/l ml resin) to which 1 mg of IgG from a non-immunized rabbit had been bound. After overnight reaction at 4 ° C, it was centrifuged to remove nonspecific binding proteins bound to the beads and the supernatant was recovered.

10 µl of the supernatant was mixed with the same quantity of the sample buffer for SDS-PAGE and 1 µl of 2-mercaptoethanol (2ME). After heat treatment at 95 ° C for 5 min, it was electrophoresed on a 5-20% gradient gel.

After electrophoresis, the gel was stained with coomassie brilliant blue. As a results, a band at the position of about 120kD was observed and the production of the recombinant h-l(3)mbt was ascertained.

Here, the calculated molecular weight of h-l(3)mbt was 86kD, and the molecular weight of the recombinant h-1(3)mbt was about 120kD.

### (Example 7)

### Preparation of Antibodies Recognizing h-l(3)mbt (1)

### 1. Preparation of Antigens

A polypeptide comprising the below-shown amino acid sequence A (set forth in SEQ ID NO:17 of the Sequence Listing) formed by appending cysteine C to the N-terminus of the amino acid sequence composed of 617th to 635th amino acid in the amino acid sequence of h-l(3)mbt set forth in SEQ ID NO: 1 of the Sequence Listing, and a polypeptide comprising the below-shown amino acid sequence B (set forth in SEQ ID NO:18 of the Sequence Listing) formed by appending cysteine C to the N-terminus of the amino acid sequence composed of 148th to 167th amino acid in the amino acid sequence of h-l(3)mbt set forth in SEQ ID NO: 1 of the Sequence Listing, were synthesized. The appending of cysteine was conducted for conjugating the peptides to hemocyanin from *Macroschisma sinensis* (KHL). The syntheses were entrusted to Iwaki Glass Co., Ltd..
Sequence A: CGRIGRPPKYRKIPQEDFQT
Sequence B: CMKKRKRREYQSPSEEESEPE

The synthesized peptide (2 mg) was conjugated to 2 mg of maleimidated KLH (hemocyanin from *Macroschisma sinensis*, available from Pierce Inc.). The reaction was carried out according to the method as described in the kit from Pierce Inc.

### 2. Immunization

100 µl of each of antigen solutions (1 µg/ml) of the antigen composed of sequence A and the antigen composed of sequence B, 0.5 ml of PBS and 0.5 ml of Freund complete adjuvant (available from Difco Inc.) were taken in a syringe and mixed to prepare an emulsion. This emulsion was subcutaneously inoculated at 4 places on the back of each rabbit.

One week later, the second immunization was carried out. The adjuvant was changed to Freund incomplete adjuvant (available from Difco Inc.) from the second time to effect immunization. The other manipulations were the same as those in the first time. After the second time, immunization was done six times in total at intervals of one week.

### 3. Purification of Antibodies

One week after the final immunization, blood was collected from the rabbits. After the collected blood was allowed to stand at room temperature for 3 h and sufficient coagulation was effected, centrifugation was done at 3,000 rpm for 5 min to recover the supernatant (serum).

This serum was salted out to bring the final concentration to 50% of saturated ammonium sulfate. This sample was centrifuged and fractions containing an antibody were precipitated. Subsequently, the precipitate was dissolved in PBS and was further dialyzed against PBS.

Then, the antibody was purified by affinity chromatography using a Protein G Sepharose column (available from Amersham Pharmacia Biotech Inc.). As a result, a total amount of 5 mg of a peptide-specific antibody was obtained.

### (Example 8)

### Preparation of Antibodies Recognizing h-l(3)mbt (2)

### 1. Preparation of Antigens

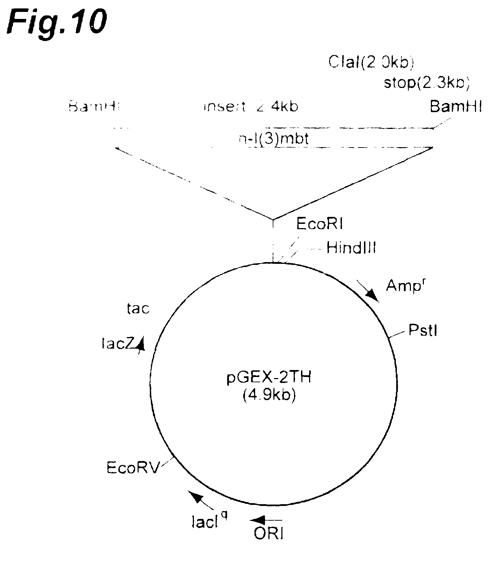

A h-l(3)mbt gene set forth in SEQ ID NO: 2 of the Sequence Listing was inserted into a vector pGEX-2TH (see Fig. 10) with a GST gene incorporated therein. This vector was introduced into to *E. coli*(DH5α) to prepare a transformant. In this transformant, GST-h-l(3)mbt was prepared by bonding GST to N-terminus of h-l(3)mbt.

The GST-bonded recombinant h-l(3)mbt was purified with the procedures as the same as those in Example 4.

The prepared peptide (2 mg) was conjugated to 2 mg of maleimidated KLH (hemocyanin from *Macroschisma sinensis*, available from Pierce Inc.). The reaction was carried out according to the method as described in the kit from Pierce Inc.

### 2. Immunization

According the procedures similar to those in Example 6, 100 µl of the antigen solution (1 µg/ml), 0.5 ml of PBS and 0.5 ml of Freund complete adjuvant (available from Difco Inc.) were taken in a syringe and mixed to prepare an emulsion. This emulsion immunized the backs of rabbits.

### 3. Purification of Antibody

An antibody from the blood of the rabbits was purified by the same process as Example 6.

### (Example 9)

### Confirmation of Reactivity of h-l(3)mbt Antibody

The h-l(3)mbt protein in COS cell was prepared according to the same procedures as those in Example 6, and the prepared protein was subjected to SDS-PAGE electrophoresis. After electrophoresis, the migrated proteins were transferred onto a nitrocellulose membrane using a TRANS BLOT SYSTEM (available from Marisol Inc.).

The nitrocellulose membrane was immersed in BLOCKACE (available from Dainippon Pharmaceutical Co. Ltd.) and left for 1 h to effect blocking. Subsequently, it was twice washed with 0.05% Tween20/PBS for 5 min.

Reactivity of each of the antibodies prepared in Examples 6 and 7 was ascertained by conducting western blotting according to the following operation: The antibody was diluted with PBS to give a concentration of 1 µg/ml, and added to the membrane to react at room temperature for 2 h. Subsequently, the membrane was three times washed with 0.05% Tween20/PBS for 5 min.

A peroxidase-labeled rabbit IgG solution (available from Caltag Inc.) was diluted with PBS 1000-fold and the solution was added to the above-described membrane and further allowed to react at room temperature for 1 h. Subsequently, the membrane was three times washed with 0.05% Tween20/PBS for 5 min.

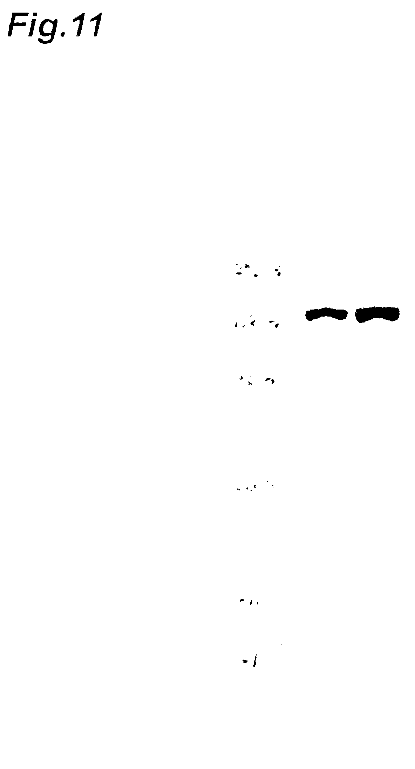

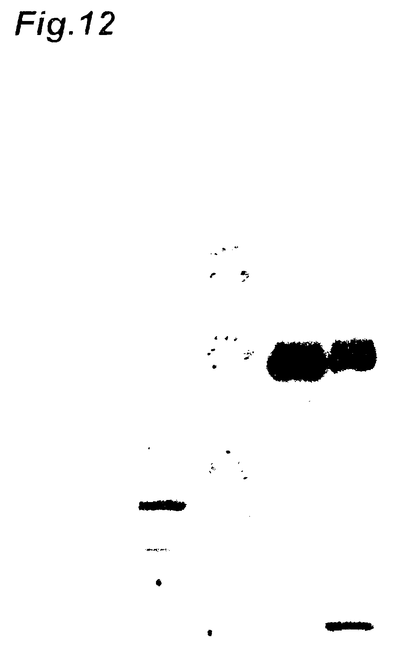

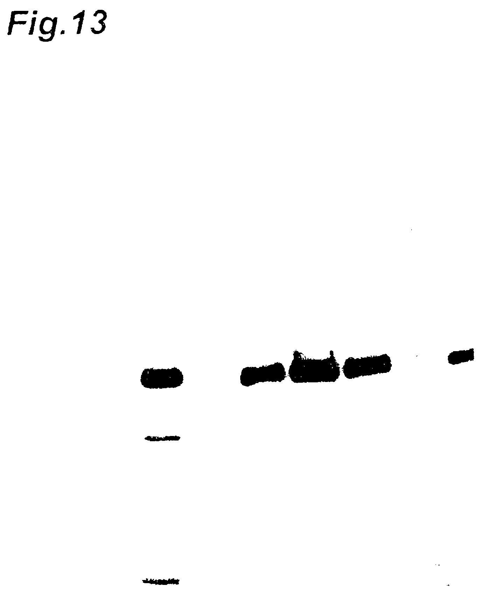

5 ml of a coloring solution from an ECL KIT (available from Amersham Pharmacia Biotech Inc.) was added to the membrane and it was allowed to react for 1 min. Then, this membrane was exposed to an X-ray film for 20 sec and the film was developed. The results are shown in Figs.11-13. As a result, a band was observed at the position of 120 kD in the reaction of any one of the antibodies, thus the ability of the antibodies to recognize h-l(3)mbt was confirmed.

### (Example 10)

### Determination of Antibody Titers

Titer of an antibody (referred to as antibody B hereinafter) obtained by immunizing a rabbit with an antigen which is the peptide comprising the amino acid sequence of sequence B prepared in Example 6 was determined by ELISA.

### 1. Preparation of antigen

The peptide composed of the amino acid sequence of sequence B was dissolved in PBS to give a concentration of 25 µg/ml, and 50µl of this solution was dispensed to each well of a 96-well ELISA plate (Xenobind, available from Xenopore Inc.), then allowed to stand overnight at 4 ° C.

The antigen solution was discarded and each 200 µl of BLOCKACE (available from Dainippon Pharmaceuticals Co. Ltd.) diluted fourfold with distilled water was dispensed to each well. Blocking was carried out by allowing it to stand at room temperature for 2 h.

### 2. Primary antibody reaction

The blocking solution was discarded and the antibody B was added as a primary antibody. A serially (in twofold) PBS-diluted solution of the antibody B was added to each well at 50 µl/well so that its concentration could consecutively reach 10 µg/ml, 5 µg/ml, 2.5 µg/ml. . .0.005 µg/ml from the first row of the 96 wells. In addition, IgG purified from the blood of a rabbit that was not immunized was used as a control.

### 3. Secondary antibody reaction

After the reaction, the antibody solution was discarded and the plate was washed with 0.05% Tween20/PBS four times. Next, each 50 µl of the biotinylated anti-rabbit IgG antibody (available from Vector Inc.) that had been 1000-fold diluted with PBS and served as a secondary antibody preparation, was dispensed to each well, and it was allowed to stand at room temperature for 1 h.

### 4. Coloring reaction

After the secondary antibody preparation was discarded, the plate was washed with 0.05% Tween20/PBS four times, and 100 µl of OPD (orthophenylenediamine)-H₂0₂/PCB was dispensed to each well. After the reaction was quenched with 2N sulfuric acid upon sufficient coloring, the absorbance at 490 nm was measured with a microplate reader (available from Bio-Rad Inc.).

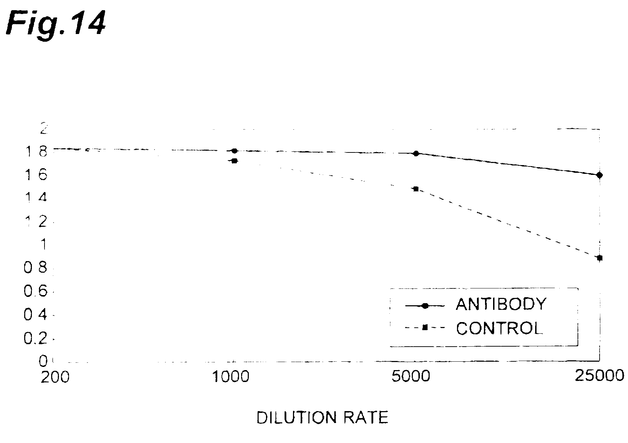

These results are shown in Fig. 14. In Fig. 14, the axis of abscissa represents concentrations of the added polyclonal antibody or the dilutions rate of the serum, and the axis of ordinate represents the absorbance at 490 nm. As shown in Fig. 14, the titer of the antibody B is greater than the control.

### (Example 11)

### Tissue Staining

The h-l(3)mbt expression pattern in T98G-MG cell (malignant glioma), in which h-l(3)mbt type 1 was expressed, was examined by immunostaining of tissues using antibody B.

The cell was cultured in Labtek chamber slide.

Next, the slide cultured cell was treated with 3.7% formaldehyde for 3 min, and then treated with methanol chilled at -20 ° C for 3 min to effect fixing. Subsequently, the slide was washed with 50 mM Tris-HCl (pH 7.5) (5 min, three times).

Subsequently BLOCKACE (available from Dainippon Pharmaceutical Co. Ltd.) was placed over the tissue on the slide glass and blocking was conducted by allowing them to stand at room temperature for 1 h. Thereafter, the slide was washed with 50 mM Tris-HCl (pH 7.5) (5 min, three times).

The antibody B was diluted with PBS to give a concentration of 1 µg/ml, and it was added onto the cell on the slide and allowed to react at room temperature for 1 h. Subsequently, the slide was washed with 50 mM Tris-HCl (pH 7.5) (5 min, three times).

A FITC-labeled anti rabbit IgG antibody solution (available from Caltag Inc.) diluted with PBS 1000-fold was added onto the cell on the slide and allowed to react at room temperature for 1 h. Subsequently, the slide was washed with 50 mM Tris-HCl (pH 7.5) (5 min, three times).

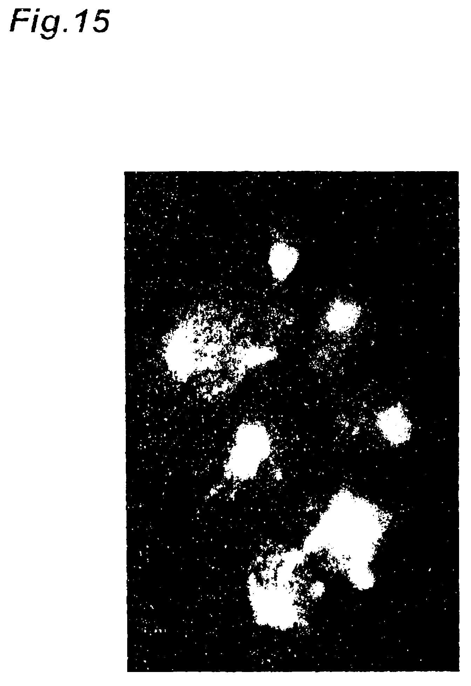

After coloring, the slide was observed under a fluorescence microscope. The microphotograph is shown in Fig. 15. As shown in Fig. 15, not only the nucleus in the cell but also the chromatin in the nucleus were colored. As a consequence, it was ascertained that l(3)mbt is the protein existing in nucleus and is localized in nucleus.

### (Example 12)

### Tissue staining of recombinant h-l(3)mbt protein

A full-length h-l(3)mbt type 2 gene was inserted into a pBJ-Myc vector, and the vector was introduced into a VA 13 cell to prepare a transformant. Further the type 1 gene was also introduced into a VA13 cell. In these transformants, the recombinant proteins were expressed. The tissue staining of nuclear DNAs in the cell was conducted with PI, and the tissue staining of the recombinant proteins was conducted with FITC.

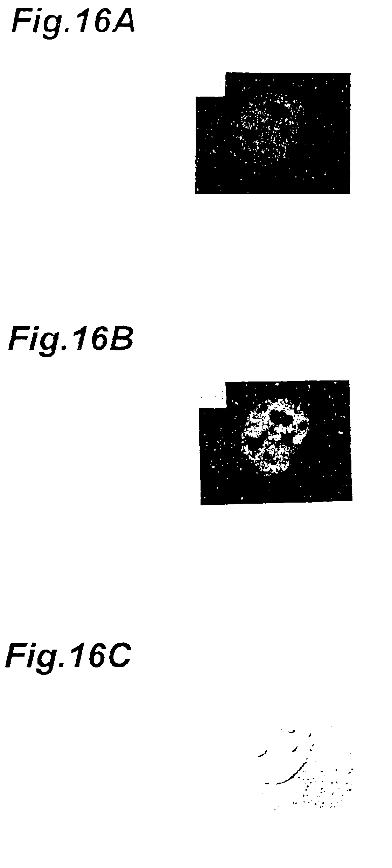

Fig. 16A is a microphotograph showing the result of PI staining of the nuclear DNA in the cell where h-l(3)mbt type 1 was expressed; Fig. 16B is a microphotograph showing the result of the staining of the h-l(3)mbt type 1 in the same cell; and Fig. 16C is a microphotograph showing the result of observation simultaneously on the nuclear DNA and the h-l(3)mbt type 1 in the cell.

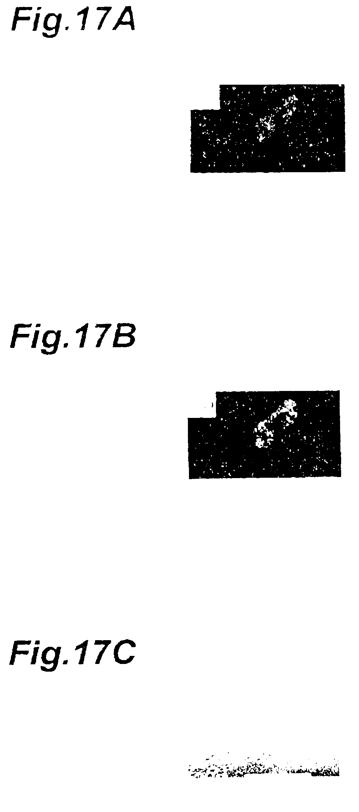

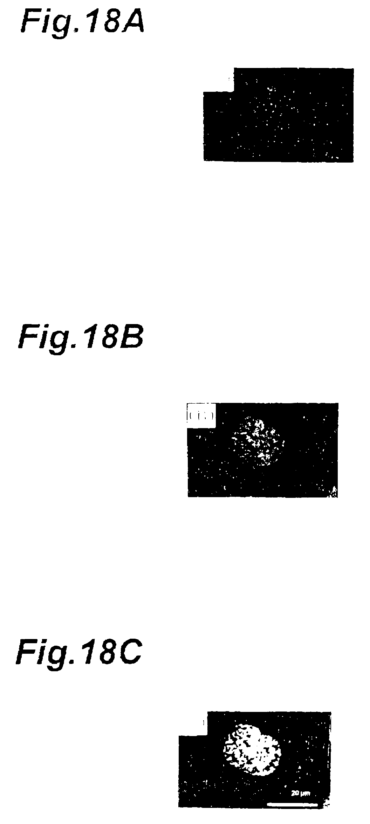

Fig. 17A is a microphotograph showing the result of PI staining of the nuclear DNA in the cell where h-l(3)mbt type 2 was expressed; Fig. 17B is a microphotograph showing the result of the staining of the h-l(3)mbt type 2 in the same cell; and Fig. 17C is a microphotograph showing the result of the observation on both h-l(3)mbt type 2 and the nuclear DNA in the cell. Fig. 18A is a microphotograph showing the result of PI staining of the nuclear DNA in a U20S cell; Fig. 18B is a microphotograph showing the result of the staining of the h-l(3) in the U20S cell; and Fig. 18C is a microphotograph showing the result of observation on both h-l(3)mbt and the nuclear DNA in the cell.

It was ascertained from these figures that, in comparison with h-l(3)mbt type 1, more h-l(3)mbt type 2 was expressed along the nuclear DNA which was agglutinated.

### (Example 13)

### Chromosomal Mapping

The chromosomal mapping of the full-length h-l(3)mbt gene was carried out. All the analysis of FISH using a part of the h-l(3)mbt gene as the probe and radiation hybrid (available from Research Genetics Inc. ))on the basis of PCR methods reached the same results, and the gene was located on chromosome 20q11.

### (Example 14)

### Expression of m-l(3)mbt in a variety of tissues

In respect to the expression of mouse l(3)mbt genes, the analysis was conducted, similar to that in Example 4, by means of northern blotting hybridization using a labeled full-length m-l(3)mbt gene and membranes (MOUSE TISSUE NORTHERN BLOT (available from Clontech Inc.) )respectively blotted with each 2µg of poly(A)+RNA (mRNA) from a variety of mouse (adult) tissues and poly(A)+RNA (mRNA) from a fetal mouse.

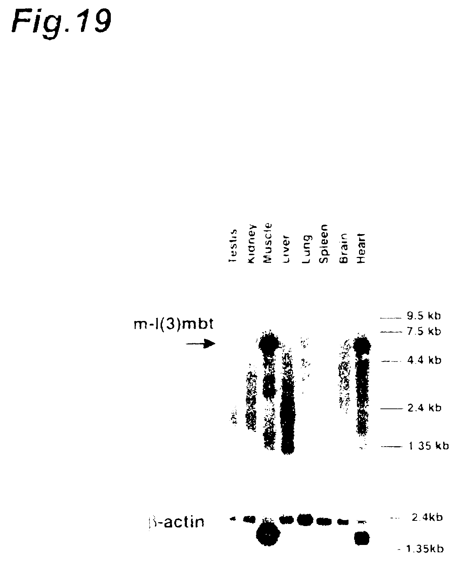

Fig. 19 is a photograph showing the results in respect to a variety of mouse adult tissues. From this photograph, m-l(3)mbt (band of 6.3kb) was recognized specific to cardiac muscle and skeletal muscle.

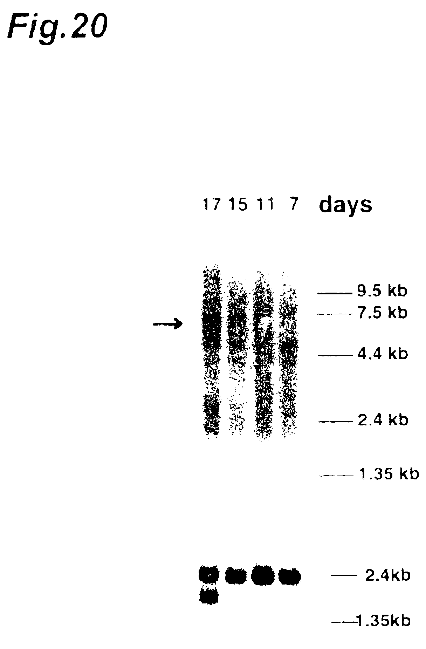

Fig. 20 is a photograph showing the results in respect to the fetal mouse. From this photograph, the expression of a band of 6.3 kb after 5 day of embryo was ascertained as well as the expression of muscle-specific b-actin.

### (Example 15)

### Preparation of Recombinant m-l(3)mbt Protein

Similar to Example 6, the m-l(3)mbt gene was introduced into a COS cell to prepare a transformant, and a recombinant m-l(3)mbt protein was prepared in said transformant.

### (Example 16)

### Preparation of antibody recognizing m-l(3)mbt

A polypeptide comprising the following-stated amino acid sequence C (set forth in SEQ ID NO:19 of the Sequence Listing) formed by appending cysteine C to the N-terminus of the amino acid sequence composed of 804th to 826th amino acids which is the C-terminus part of the amino acid sequence of m-l(3)mbt set forth in SEQ ID NO: 7 of the Sequence Listing was synthesized. The antibody recognizing m-l(3)mbt was prepared in the same manner as that in Example 8.
Sequence C: KLGPALKIYNAILMFKNNDDVFK

### (Example 17)

### Confirmation of reactivity of m-l(3)mbt antibody

In the manner similar to that of Example 10, the m-l(3)mbt protein prepared in COS cell was subjected to SDS-PAGE electrophoresis. After electrophoresis, the migrated proteins were transferred onto a nitrocellulose membrane by using a TRANS BLOT SYSTEM (available from Marisol Inc.).

The nitrocellulose membrane was immersed in BLOCKACE (available from Dainippon Pharmaceutical Co. Ltd.) and left for 1 h to effect blocking. Subsequently, it was twice washed with 0.05% Tween20/PBS for 5 min.

The reactivities of the anti-Myc antibody and the antibody prepared in Example 16 were confirmed by means of western blotting hybridization as the same as that in Example 9.

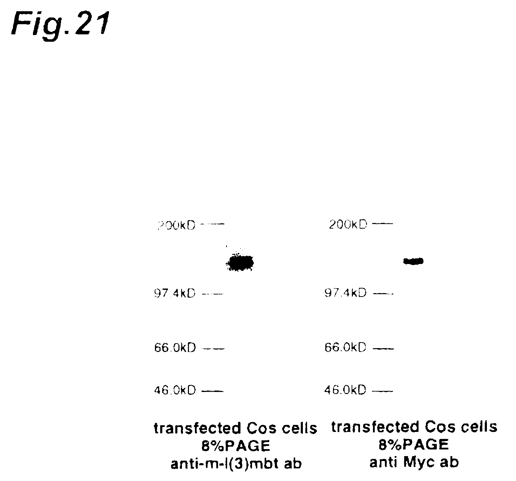

The results are shown in Fig. 21. As shown in Fig. 21, a band which has as same size as that of human being (the position of 120kD) substantially the same as that of human beings was observed and the ability of the antibody prepared in Example 16 to recognize m-l(3)mbt was ascertained as the results of subjecting the antibodies to the reaction.

### (Example 18)

### Tissue Staining of m-l(3)mbt

Mouse cell line SWISS 3T3 was stained with the antibody prepared in Example 16 in the manner similar to Example 12.

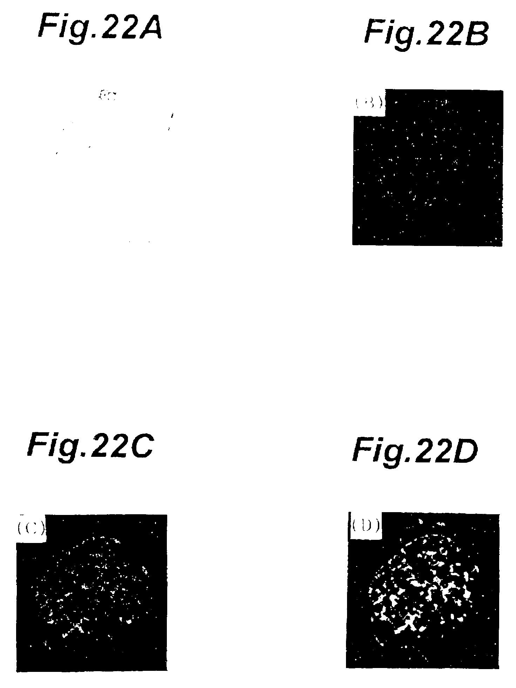

Figs. 22A-D are photographs showing the results. Fig. 22A is a microphotograph of a cell; Fig. 22B shows the result of the staining of the m-l(3)mbt in the same cell that shown in Fig. 22A; and Fig. 22C shows the result of PI staining of DNA in the same cell that shown in Figs. 22A and B; and Fig. 22D shows the results of superposition of Fig. 22A and Fig. 22B. As shown in the photographs, the nucleic chromatin was stained.

### (Example 19)

### Cellular transformation caused by the expression of l(3)mbt genes

l(3)mbt was expressed with Cre/LoxP system, and the transformation of cellular morphology was observed. The information in respect to Cre/LoxP system can be obtained from an internet homepage (http://www.ims.u-tokyo.ac.jp/idenshi/hpmain.html) of The Laboratory of Molecular Genetics of the Insitute of Medical Science, the University of Tokyo.

h-l(3)mbt type 1 cDNA and type 2 cDNA were incorporated to a cassette plasmid pCALNLw (assigned by The Laboratory of Molecular Genetics of the Insitute of Medical Science, the University of Tokyo) for ON/OFF expression, and were respectively introduced into VA13 and U251 cell lines, where the expression of h-l(3)mbt was not recognized, to obtain stable expression cell line. The cell lines were infected with Cre recombinase expression adenovirus, and the transformation of cellular morphology was observed. As a result, the expression of multinucleate cell was recognized in these two kinds of cell lines and both case of type 1 and type 2.

It was clarified from this result that l(3)mbt genes and l(3)mbt are involved in cell division, and the expression of l(3)mbt is a significant step in carcinogenesis.

### INDUSTRIAL APPLICABILITY

l(3)mbt, l(3)mbt genes and antibodies recognizing l(3)mbt of the present invention are effective in analysing the formation mechanism of malignant turmors.

Further, l(3)mbt genes and l(3)mbt are the substances involved in intranuclear transcription and cell division.

Sequence Listing
SEQ ID NO: 1
Sequence Length: 772
Sequence Type: amino acid
Topology: straight chain
Kind of Sequence: peptide
Sequence:
Met Arg Arg Arg Glu Gly His Gly Thr Asp Ser Glu Met Gly Gln Gly
1               5                   10                  15
Pro Val Arg Glu Ser Gln Ser Ser Asp Pro Pro Ala Leu Gln Phe Arg
            20                  25                  30
Ile Ser Glu Tyr Lys Pro Leu Asn Met Ala Gly Val Glu Gln Pro Pro
        35                  40                  45
Ser Pro Glu Leu Arg Gln Glu Gly Val Thr Glu Tyr Glu Asp Gly Gly
    50                  55                  60
Ala Pro Ala Gly Asp Gly Glu Ala Gly Pro Gln Gln Ala Glu Asp His
65                  70                  75                  80
Pro Gln Asn Pro Pro Glu Asp Pro Asn Gln Asp Pro Pro Glu Asp Asp
                85                  90                  95
Ser Thr Cys Gln Cys Gln Ala Cys Gly Pro His Gln Ala Ala Gly Pro
            100                 105                 110
Asp Leu Gly Ser Ser Asn Asp Gly Cys Pro Gln Leu Phe Gln Glu Arg
        115                 120                 125
Ser Val Ile Val Glu Asn Ser Ser Gly Ser Thr Ser Ala Ser Glu Leu
    130                 135                 140
Leu Lys Pro Met Lys Lys Arg Lys Arg Arg Glu Tyr Gln Ser Pro Ser

145                 150                 155                 160
Glu Glu Glu Ser Glu Pro Glu Ala Met Glu Lys Gln Glu Glu Gly Lys
                165                 170                 175
Asp Pro Glu Gly Gln Pro Thr Ala Ser Thr Pro Glu Ser Glu Glu Trp
            180                 185                 190
Ser Ser Ser Gln Pro Ala Thr Gly Glu Lys Lys Glu Cys Trp Ser Trp
        195                 200                 205
Glu Ser Tyr Leu Glu Glu Gln Lys Ala Ile Thr Ala Pro Val Ser Leu
    210                 215                 220
Phe Gln Asp Ser Gln Ala Val Thr His Asn Lys Asn Gly Phe Lys Leu
225                 230                 235                 240
Gly Met Lys Leu Glu Gly Ile Asp Pro Gln His Pro Ser Met Tyr Phe
                245                 250                 255
Ile Leu Thr Val Ala Glu Val Cys Gly Tyr Arg Leu Arg Leu His Phe
            260                 265                 270
Asp Gly Tyr Ser Glu Cys His Asp Phe Trp Val Asn Ala Asn Ser Pro
        275                 280                 285
Asp Ile His Pro Ala Gly Trp Phe Glu Lys Thr Gly His Lys Leu Gln
29[?] 295 300
Leu Pro Lys Gly Tyr Lys Glu Glu Glu Phe Ser Trp Ser Gln Tyr Met
305 310 315 320
Cys Ser Thr Arg Ala Gln Ala Ala Pro Lys His Met Phe Val Ser Gln
325 330 335
Ser His Ser Pro Pro Pro Leu Gly Phe Gln Val Gly Met Lys Leu Glu
340 345 350
Ala Val Asp Arg Met Asn Pro Ser Leu Val Cys Val Ala Ser Val Thr

355 360 365

Asp Val Val Asp Ser Arg Phe Leu Val His Phe Asp Asn Trp Asp Asp

370 375 380

Thr Tyr Asp Tyr Trp Cys Asp Pro Ser Ser Pro Tyr Ile His Pro Val

385 390 395 400

Gly Trp Cys Gln Lys Gln Gly Lys Pro Leu Thr Pro Pro Gln Asp Tyr

405 410 415

Pro Asp Pro Asp Asn Phe Cys Trp Glu Lys Tyr Leu Glu Glu Thr Gly

420 425 430

Ala Ser Ala Val Pro Thr Trp Ala Phe Lys Val Arg Pro Pro His Ser

435 440 445

Phe Leu Val Asn Met Lys Leu Glu Ala Val Asp Arg Arg Asn Pro Ala

450 455 460

Leu Ile Arg Val Ala Ser Val Glu Asp Val Glu Asp His Arg Ile Lys

465 470 475 480

Ile His Phe Asp Gly Trp Ser His Gly Tyr Asp Phe Trp Ile Asp Ala

485 490 495

Asp His Pro Asp Ile His Pro Ala Gly Trp Cys Ser Lys Thr Gly His

500 505 510

Pro Leu Gln Pro Pro Leu Gly Pro Arg Glu Pro Ser Ser Ala Ser Pro

515 520 525

Gly Gly Cys Pro Pro Leu Ser Tyr Arg Ser Leu Pro His Thr Arg Thr

530 535 540

Ser Lys Tyr Ser Phe His His Arg Lys Cys Pro Thr Pro Gly Cys Asp

545 550 555 560

Gly Ser Gly His Val Thr Gly Lys Phe Thr Ala His His Cys Leu Ser

565 570 575
Gly Cys Pro Leu Ala Glu Arg Asn Gln Ser Arg Leu Lys Ala Glu Leu
580 585 590
Ser Asp Pro Glu Ala Ser Ala Arg Lys Lys Asn Leu Ser Gly Phe Ser
595 600 605
Pro Arg Lys Lys Pro Arg His His Gly Arg Ile Gly Arg Pro Pro Lys
610 615 620
Tyr Arg Lys Ile Pro Gln Glu Asp Phe Gln Thr Leu Thr Pro Asp Val
625 630 635 640
Val His Gln Ser Leu Phe Met Ser Ala Leu Ser Ala His Pro Asp Arg
645 650 655
Ser Leu Ser Val Cys Trp Glu Gln His Cys Lys Leu Leu Pro Gly Val
660 665 670
Ala Gly Ile Ser Ala Ser Thr Val Ala Lys Trp Thr Ile Asp Glu Val
675 680 685
Phe Gly Phe Val Gln Thr Leu Thr Gly Cys Glu Asp Gln Ala Arg Leu
690 695 700
Phe Lys Asp Glu Ala Arg Ile Val Arg Val Thr His Val Ser Gly Lys
705 710 715 720
Thr Leu Val Trp Thr Val Ala Gln Leu Gly Asp Leu Val Cys Ser Asp
725 730 735
His Leu Gln Glu Gly Lys Gly Ile Leu Gly Thr Gly Val His Ser Leu
740 745 750
Leu Cys Ser Leu Pro Thr His Leu Leu Ala Lys Leu Ser Phe Ala Ser
755 760 765
Asp Ser Gln Tyr

770

SEQ ID NO: [?]
Sequence Length 3394
Sequence Type: nucleic acid
Strandedness: double
Topology: straight chain
Kind of Sequence: cDNA to mRNA
Sequence:
ACATAGAGAT AAAGGGAATG AACTATAAAG ACACTGGCAT CTCTGTTTCC CACAAATCAA 60
GACATTCATG AAATTAATGA CTAACTTACA ATGAATTTTT TAGAGGTGTG TCTCATAAGG 120
AAAAACCTGG ATGATATGGG ACCCCGCAGG TGATTGGGGG CGTAGCCTGG AGTGAGGGTT 180
TGGCTGGTGT AGCTTGGAGT GAGGCCCCCT GGCGTGGAGT CTTGAGGCCT GCTGAGGGCG 240
TGAGATGGC ATG AGG CGA AGA GAG GGC CAT GGC ACC GAC TCC GAG ATG GGT 291
CAA GGA CCC GTA CGG GAG TCG CAA TCC TCA GAC CCT CCC GCG CTC CAG 339
TTC CGG ATA AGC GAG TAT AAG CCG CTG AAC ATG GCG GGA GTG GAG CAG 387
CCC CCG AGC CCC GAG CTG CGG CAG GAA GGC GTG ACC GAA TAC GAA GAT 435
GGC GGG GCC CCG GCG GGA GAT GGC GAG GCG GGC CCC CAA CAG GCG GAG 483
GAC CAC CCC CAG AAT CCT CCA GAA GAT CCC AAT CAG GAC CCC CCA GAG 531
GAT GAT AGC ACC TGT CAG TGC CAG GCG TGC GGG CCT CAC CAA GCC GCG 579
GGT CCA GAT CTT GGT TCC TCT AAT GAT GGC TGC CCT CAG CTG TTC CAG 627
GAG CGG TCA GTC ATA GTG GAG AAC TCC TCA GGC TCT ACC AGC GCT TCT 675
GAG CTC CTC AAA CCC ATG AAG AAG AGG AAG CGC AGG GAA TAC CAG AGC 723
CCA TCA GAG GAG GAG TCG GAG CCA GAG GCC ATG GAG AAG CAA GAA GAA 771
GGA AAG GAC CCA GAG GGA CAA CCC ACT GCT AGC ACC CCA GAG AGT GAG 819
GAG TGG AGC AGC AGC CAG CCT GCA ACA GGT GAG AAG AAG GAA TGC TGG 867

TCG TGG GAG T[?][?] TA[?] CTA GAG GAG CAG AAG GCC ATT ACT GCT CCA GTC 915

AGC [?]TC [?][?]C CAG GA[?] TCC CAG GCA GTC ACT CAC AAC AAG AAT GGC TTC 963

AAA CTG GGC ATG AAG TTG GAA GGC ATT GAC CCT CAA CAC CCG TCC ATG 1011

TAC TTC ATC CTC ACC GTG GCT GAG GTA TGT GGC TAT CGC CTA CGC CTG 1059

CAC TTT GAT GGG TAT TCT GAG TGC CAT GAC TTC TGG GTC AAT GCC AAC 1107

TCC CCT GAC ATT CAC CCT GCT GGC TGG TTC GAG AAG ACG GGC CAC AAG 1155

CTG CAG CTT CCC AAA GGT TAC AAG GAG GAG GAG TTC AGC TGG AGC CAG 1203

TAC ATG TGC AGC ACA AGA GCT CAG GCT GCC CCC AAG CAC ATG TTT GTG 1251

AGC CAG AGC CAC AGT CCC CCA CCC TTG GGC TTC CAG GTG GGC ATG AAG 1299

CTG GAG GCT GTT GAC CGC ATG AAC CCG TCC CTT GTC TGC GTG GCC AGT 1347

GTG ACC GAT GTG GTG GAC AGC CGC TTC CTG GTG CAC TTT GAC AAC TGG 1395

GAT GAT ACT TAT GAC TAC TGG TGT GAT CCC AGC AGC CCC TAC ATC CAC 1443

CCA GTG GGC TGG TGC CAG AAG CAA GGA AAG CCC CTC ACC CCT CCA CAA 1491

GAC TAC CCA GAC CCT GAT AAC TTC TGT TGG GAG AAA TAT CTG GAA GAA 1539

ACT GGG GCC TCT GCT GTC CCC ACC TGG GCC TTC AAG GTG CGA CCC CCT 1587

CAC AGC TTC CTG GTC AAT ATG AAG CTG GAG GCT GTG GAC CGC AGG AAC 1635

CCA GCC CTG ATT CGC GTG GCC AGC GTG GAG GAT GTG GAG GAC CAT CGG 1683

ATA AAG ATC CAC TTT GAT GGC TGG AGT CAT GGC TAT GAT TTC TGG ATC 1731

GAC GCT GAT CAC CCA GAC ATC CAC CCT GCC GGC TGG TGC TCC AAG ACA 1779

GGA CAT CCC CTG CAG CCT CCT CTC GGA CCC AGA GAG CCC AGC TCT GCC 1827

TCC CCT GGG GGC TGT CCC CCT CTC AGC TAT AGG AGC CTG CCC CAC ACT 1875

AGG ACC TCC AAA TAC AGC TTT CAC CAC CGG AAG TGC CCC ACT CCT GGT 1923

TGC GAC GGC TCT GGC CAT GTC ACA GGC AAG TTC ACA GCT CAC CAT TGC 1971

CTC TCA GGC TGC CCA CTG GCT GAG AGG AAC CAG AGC CGG CTG AAA GCG 2019

GAG CTG TCT GAC CCG GAG GCC TCA GCC CGC AAG AAG AAC CTC TCA GGC 2067

TTC TCC CCA AGG AAG AAG CCT CGC CAT CAC GGC CGA ATT GGA CGC CCT 2115

CCG AAG TA[?] [?]GA AA[?] AT[?] [?][?]G [?]AG GAA GA[?] T[?]C CAG ACC CTC ACG CCC 2163

GAT GTC [?][?]G [?]A[?] CA[?] [?][?]C [?]T[?] [?][?][?] A[?]G TCA GCC CTG TCG GCC CAC CCT 2211

GAC CGC [?][?]A [?]T[?] [?]CA GTG [?]GC [?]GG GAG CAG CAC TGC AAG CTC CTG CCA 2259

GGA GTA [?]CG G[?]C AT[?] [?]CA G[?]C TC[?] ACA GTC GCC AAG TGG ACC ATC GAT 2307

GAG GTC [?][?]C GGC TT[?] GT[?] CAG AC[?] C[?]G ACA GGT TGT GAG GAC CAA GCA 2355

CGC CTC [?][?]C AAA GAC GAG GCA AGA ATA GTC AGA GTG ACC CAT GTA TCT 2403

GGG AAG ACT CTA GTC [?]GG ACT G[?]G GCC CAG CTT GGG GAC CTT GTG TGC 2451

TCA GAT CA[?] C[?]T CAG GAA GGA AAA GGC ATC CTG GAG ACA GGA GTC CAT 2499

TCA CTC CTC TGC TCT CTA CCC AC[?] CAT TTG CTT GCC AAA CTT AGC TTT 2547

GCC AGT GAT AGT CAA TAT TAA AGTGTACTTT TTTCCCCTTT AATCCAATAT 2598

AGTTGATAAT TAAAGTGTAT TTTGAATGAC ACAGATATTG TGATTTACTG CAAGGATCCT 2658

AACACACACT TAAAATCAAG AGCCAAGGAG TAGTGAGTTG TAGATAAAAA AAGAATGTCA 2718

GCTTTGGAGA CAGTCTGGGT TTAAATCCCA GTTCTGTCAA TTTGAGCTGT TTACTGTCTC 2778

TGAGCCTACA TCTTCTTGTC TGTAAAATGG AGATAAAATG GGTTTAATGA GGTCTACCTT 2838

GCAGAGCCAT TGTGAGCATT GGAAATGATG AATGAATCAT ACCAGAACGT CTAGTATAAT 2898

TACAGTCATG CATTGCTTAA CGATGGGGAT ACATTCTTAG AAATGTGTCA CTAGGCAATT 2958

CTGTCATTGT GTAAACATTA TAGGATGTAC TTACACAAAC CTAGATGTTA TATGTATTTT 3018

TATTTACATG TATATTTTCA CATGAAATAC CAAATGTCAC AGCATTATTA CTGAATGTCA 3078

GTCATTTCCC CTACTTGATC TGCAATGCCA ATATCAAGGG CCATGTATCA GGTTTCTGTA 3138

TATGTTCCAC TATAATCTTA TGGGACCATG GTTTTAAATG TGGAATCATT GACAGAAATG 3198

TNTTTATGTA GCATATGGCT GTGTATCACT AGTATATAAT AGAGCAATAT TATGGAGGAA 3258

TATGTAGATC CAATCACTTT ACCTATACAA AATGACTGCT ATGGTGGGAA CACAATAAAC 3318

ACCAGTTTTG ACTTTTAAAA AAAAAAAAAA CAAAAAAAAA AAAAAAAAGC GGCCGCTGAA 3378

TTCACCTGCC CGGGCG 3394

SEQ ID NO: 3

Sequence Length: 738
Sequence Type: amino acid
Topology: straight chain
Kind of Sequence: peptide
Sequence:
Met Arg Arg Arg Glu Gly His Gly Thr Asp Ser Glu Met Gly Gln Gly
1               5                   10                  15
Pro Val Arg Glu Ser Gln Ser Ser Asp Pro Pro Ala Leu Gln Phe Arg
            20                  25                  30
Ile Ser Glu Tyr Lys Pro Leu Asn Met Ala Gly Val Glu Gln Pro Pro
        35                  40                  45
Ser Pro Glu Leu Arg Gln Glu Gly Val Thr Glu Tyr Glu Asp Gly Gly
    50                  55                  60
Ala Pro Ala Gly Asp Gly Glu Ala Gly Pro Gln Gln Ala Glu Asp His
65                  70                  75                  80
Pro Gln Asn Pro Pro Glu Asp Pro Asn Gln Asp Pro Pro Glu Asp Asp
                85                  90                  95
Ser Thr Cys Gln Cys Gln Ala Cys Gly Pro His Gln Ala Ala Gly Pro
            100                 105                 110
Asp Leu Gly Ser Ser Asn Asp Gly Cys Pro Gln Leu Phe Gln Glu Arg
        115                 120                 125
Ser Val Ile Val Glu Asn Ser Ser Gly Ser Thr Ser Ala Ser Glu Leu
    130                 135                 140
Leu Lys Pro Met Lys Lys Arg Lys Arg Arg Glu Tyr Gln Ser Pro Ser
145                 150                 155                 160
Glu Glu Glu Ser Glu Pro Glu Ala Met Glu Lys Gln Glu Glu Gly Lys

                165                 170                 175
Asp Pro Glu Gly Gln Pro Thr Ala Ser Thr Pro Glu Ser Glu Glu Trp
            180                 185                 190
Ser Ser Ser Gln Pro Ala Thr Gly Glu Lys Lys Glu Cys Trp Ser Trp
        195                 200                 205
Glu Ser Tyr Leu Glu Glu Gln Lys Ala Ile Thr Ala Pro Val Ser Leu
    210                 215                 220
Phe Gln Asp Ser Gln Ala Val Thr His Asn Lys Asn Gly Phe Lys Leu
225                 230                 235                 240
Gly Met Lys Leu Glu Gly Ile Asp Pro Gln His Pro Ser Met Tyr Phe
                245                 250                 255
Ile Leu Thr Val Ala Glu Val Cys Gly Tyr Arg Leu Arg Leu His Phe
            260                 265                 270
Asp Gly Tyr Ser Glu Cys His Asp Phe Trp Val Asn Ala Asn Ser Pro
        275                 280                 285
Asp Ile His Pro Ala Gly Trp Phe Glu Lys Thr Gly His Lys Leu Gln
    290                 295                 300
Leu Pro Lys Gly Tyr Lys Glu Glu Glu Phe Ser Trp Ser Gln Tyr Met
305                 310                 315                 320
Cys Ser Thr Arg Ala Gln Ala Ala Pro Lys His Met Phe Val Ser Gln
                325                 330                 335
Ser His Ser Pro Pro Pro Leu Gly Phe Gln Val Gly Met Lys Leu Glu
            340                 345                 350
Ala Val Asp Arg Met Asn Pro Ser Leu Val Cys Val Ala Ser Val Thr
        355                 360                 365
Asp Val Val Asp Ser Arg Phe Leu Val His Phe Asp Asn Trp Asp Asp

    370                 375                 380
Thr Tyr Asp Tyr Trp Cys Asp Pro Ser Ser Pro Tyr Ile His Pro Val
385                 390                 395                 400
Gly Trp Cys Gln Lys Gln Gly Lys Pro Leu Thr Pro Pro Gln Asp Tyr
                405                 410                 415
Pro Asp Pro Asp Asn Phe Cys Trp Glu Lys Tyr Leu Glu Glu Thr Gly
            420                 425                 430
Ala Ser Ala Val Pro Thr Trp Ala Phe Lys Val Arg Pro Pro His Ser
        435                 440                 445
Phe Leu Val Asn Met Lys Leu Glu Ala Val Asp Arg Arg Asn Pro Ala
    450                 455                 460
Leu Ile Arg Val Ala Ser Val Glu Asp Val Glu Asp His Arg Ile Lys
465                 470                 475                 480
Ile His Phe Asp Gly Trp Ser His Gly Tyr Asp Phe Trp Ile Asp Ala
                485                 490                 495
Asp His Pro Asp Ile His Pro Ala Gly Trp Cys Ser Lys Thr Gly His
            500                 505                 510
Pro Leu Gln Pro Pro Leu Gly Pro Arg Glu Pro Ser Ser Ala Ser Pro
        515                 520                 525
Gly Gly Cys Pro Pro Leu Ser Tyr Arg Ser Leu Pro His Thr Arg Thr
    530                 535                 540
Ser Lys Tyr Ser Phe His His Arg Lys Cys Pro Thr Pro Gly Cys Asp
545                 550                 555                 560
Gly Ser Gly His Val Thr Gly Lys Phe Thr Ala His His Cys Leu Ser
                565                 570                 575
Gly Cys Pro Leu Ala Glu Arg Asn Gln Ser Arg Leu Lys Ala Glu Leu

580                585                590

Ser Asp Pro Glu Ala Ser Ala Arg Lys Lys Asn Leu Ser Gly Phe Ser

        595                600                605

Pro Arg Lys Lys Pro Arg His His Gly Arg Ile Gly Arg Pro Pro Lys

    610                615                620

Tyr Arg Lys Ile Pro Gln Glu Asp Phe Gln Thr Leu Thr Pro Asp Val

625                630                635                640

Val His Gln Ser Leu Phe Met Ser Ala Leu Ser Ala His Pro Asp Arg

                645                650                655

Ser Leu Ser Val Cys Trp Glu Gln His Cys Lys Leu Leu Pro Gly Val

            660                665                670

Ala Gly Ile Ser Ala Ser Thr Val Ala Lys Trp Thr Ile Asp Glu Val

        675                680                685

Phe Gly Phe Val Gln Thr Leu Thr Gly Cys Glu Asp Gln Ala Arg Leu

    690                695                700

Phe Lys Asp Glu Val Arg Cys Lys Cys Arg Val Gly Asp Arg Ala Gly

705                710                715                720

Val Thr Val Leu Lys Thr Ala Gly Ser Arg Cys Pro Pro Gln Arg His

                725                730                735

Leu Cys

SEQ ID NO: 4

Sequence Length: 3512

Sequence Type: nucleic acid

Strandedness: double

Topology: straight chain

Kind of Sequence: cDNA to mRNA

Sequence:

ACATAGAGAT AAAGGGAATG AACTATAAAG ACACTGGCAT CTCTGTTTCC CACAAATCAA 60

GACATTCATG AAATTAATGA CTAACTTACA ATGAATTTTT TAGAGGTGTG TCTCATAAGG 120

AAAAACCTGG ATGATATGGG ACCCCGCAGG TGATTGGGGG CGTAGCCTGG AGTGAGGGTT 180

TGGCTGGTGT AGCTTGGAGT GAGGCCCCCT GGCGTGGAGT CTTGAGGCCT GCTGAGGGCG 240

TGAGATGGC ATG AGG CGA AGA GAG GGC CAT GGC ACC GAC TCC GAG ATG GGT 291

CAA GGA CCC GTA CGG GAG TCG CAA TCC TCA GAC CCT CCC GCG CTC CAG 339

TTC CGG ATA AGC GAG TAT AAG CCG CTG AAC ATG GCG GGA GTG GAG CAG 387

CCC CCG AGC CCC GAG CTG CGG CAG GAA GGC GTG ACC GAA TAC GAA GAT 435

GGC GGG GCC CCG GCG GGA GAT GGC GAG GCG GGC CCC CAA CAG GCG GAG 483

GAC CAC CCC CAG AAT CCT CCA GAA GAT CCC AAT CAG GAC CCC CCA GAG 531

GAT GAT AGC ACC TGT CAG TGC CAG GCG TGC GGG CCT CAC CAA GCC GCG 579

GGT CCA GAT CTT GGT TCC TCT AAT GAT GGC TGC CCT CAG CTG TTC CAG 627

GAG CGG TCA GTC ATA GTG GAG AAC TCC TCA GGC TCT ACC AGC GCT TCT 675

GAG CTC CTC AAA CCC ATG AAG AAG AGG AAG CGC AGG GAA TAC CAG AGC 723

CCA TCA GAG GAG GAG TCG GAG CCA GAG GCC ATG GAG AAG CAA GAA GAA 771

GGA AAG GAC CCA GAG GGA CAA CCC ACT GCT AGC ACC CCA GAG AGT GAG 819

GAG TGG AGC AGC AGC CAG CCT GCA ACA GGT GAG AAG AAG GAA TGC TGG 867

TCG TGG GAG TCC TAC CTA GAG GAG CAG AAG GCC ATT ACT GCT CCA GTC 915

AGC CTC TTC CAG GAC TCC CAG GCA GTC ACT CAC AAC AAG AAT GGC TTC 963

AAA CTG GGC ATG AAG TTG GAA GGC ATT GAC CCT CAA CAC CCG TCC ATG 1011

TAC TTC ATC CTC ACC GTG GCT GAG GTA TGT GGC TAT CGC CTA CGC CTG 1059

CAC TTT GAT GGG TAT TCT GAG TGC CAT GAC TTC TGG GTC AAT GCC AAC 1107

TCC CCT GAC ATT CAC CCT GCT GGC TGG TTC GAG AAG ACG GGC CAC AAG 1155

CTG CAG CTT CCC AAA GGT TAC AAG GAG GAG GAG TTC AGC TGG AGC CAG 1203

TAC ATG TG[?] AG[?] A[?]A AGA GCT CAG [?]C[?] GCC CCC AAG CAC ATG TTT GTG 1251

AGC CAG AG[?] CAC AGT [?]CC CCA [?]CC T[?]G GGC TTC CAG GTG GGC ATG AAG 1299

CTG GAG GCT GT[?] GAC [?]GC ATG AAC CCG TCC CTT GTC TGC GTG GCC AGT 1347

[?]TG ACC GA[?] G[?]G G[?]G [?]AC AGC C[?]C [?]TC C[?]G GTG CAC TTT GAC AAC TGG 1395

GAT GAT AC[?] TAT GA[?] [?]AC TGG TGT GA[?] CCC AGC AGC CCC [?]AC ATC CAC 1443

CCA GTG GG[?] TGG TG[?] [?]AG AAG CAA GGA AAG CCC CTC ACC CCT CCA CAA 1491

GAC TAC CCA GAC CCT GAT AAC TTC TGT TGG GAG AAA TAT CTG GAA GAA 1539

ACT GGG GCC TCT GCT GTC CCC ACC TGG GCC TTC AAG GTG CGA CCC CCT 1587

CAC AGC TTC CTG GTC AAT ATG AAG CTG GAG GCT GTG GAC CGC AGG AAC 1635

CCA GCC CTG ATT CGC GTG GCC AGC GTG GAG GAT GTG GAG GAC CAT CGG 1683

ATA AAG ATC CAC TTT GAT GGC TGG AGT CAT GGC TAT GAT TTC TGG ATC 1731

GAC GCT GAT CAC CCA GAC ATC CAC CCT GCC GGC TGG TGC TCC AAG ACA 1779

GGA CAT CCC CTG CAG CCT CCT CTC GGA CCC AGA GAG CCC AGC TCT GCC 1827

TCC CCT GGG GGC TGT CCC CCT CTC AGC TAT AGG AGC CTG CCC CAC ACT 1875

AGG ACC TCC AAA TAC AGC TTT CAC CAC CGG AAG TGC CCC ACT CCT GGT 1923

TGC GAC GGC TCT GGC CAT GTC ACA GGC AAG TTC ACA GCT CAC CAT TGC 1971

CTC TCA GGC TGC CCA CTG GCT GAG AGG AAC CAG AGC CGG CTG AAA GCG 2019

GAG CTG TCT GAC CCG GAG GCC TCA GCC CGC AAG AAG AAC CTC TCA GGC 2067

TTC TCC CCA AGG AAG AAG CCT CGC CAT CAC GGC CGA ATT GGA CGC CCT 2115

CCG AAG TAT CGA AAG ATT CCG CAG GAA GAT TTC CAG ACC CTC ACG CCC 2163

GAT GTC GTG CAC CAG TCC CTC TTC ATG TCA GCC CTG TCG GCC CAC CCT 2211

GAC CGC TCA CTC TCA GTG TGC TGG GAG CAG CAC TGC AAG CTC CTG CCA 2259

GGA GTA GCG GGC ATC TCA GCC TCG ACA GTC GCC AAG TGG ACC ATC GAT 2307

GAG GTC TTC GGC TTT GTT CAG ACC CTG ACA GGT TGT GAG GAC CAA GCA 2355

CGC CTC TTC AAA GAC GAG GTA AGG TGC AAG TGC AGA GTG GGA GAC AGA 2403

GCC GGG GTC ACT GTC CTT AAG ACG GCA GGA AGC AGG TGC CCT CCC CAG 2451

[?]GT CAC [?]TG [?]G[?] [?]AA GGAGGGACCC GTGTTGCCCC ACCAGGCAAG AATAGTCAGA 2506

GTGACCCATG TATCTGGGAA GACTCTAGTC TGGACTGTGG CCCAGCTTGG GGACCTTGTG 2566

TGCTCAGATC ATCTTCAGGA AGGAAAAGGC ATCCTGGAGA CAGGAGTCCA TTCACTCCTC 2626

TGCTCTCTAC CCACTCATTT GCTTGCCAAA CTTAGCTTTG CCAGTGATAG TCAATATTAA 2686

AGTGTACTTT TTTCCCCTTT AATCCAATAT AGTTGATAAT TAAAGTGTAT TTTGAATGAC 2746

ACAGATATTG TGATTTACTG CAAGGATCCT AACACACACT TAAAATCAAG AGCCAAGGAG 2806

TAGTGAGTTG TAGATAAAAA AAGAATGTCA GCTTTGGAGA CAGTCTGGGT TTAAATCCCA 2866

GTTCTGTCAA TTTGAGCTGT TTACTGTCTC TGAGCCTACA TCTTCTTGTC TGTAAAATGG 2926

AGATAAAATG GGTTTAATGA GGTCTACCTT GCAGAGCCAT TGTGAGCATT GGAAATGATG 2986

AATGAATCAT ACCAGAACGT CTAGTATAAT TACAGTCATG CATTGCTTAA CGATGGGGAT 3046

ACATTCTTAG AAATGTGTCA CTAGGCAATT CTGTCATTGT GTAAACATTA TAGGATGTAC 3106

TTACACAAAC CTAGATGTTA TATGTATTTT TATTTACATG TATATTTTCA CATGAAATAC 3166

CAAATGTCAC AGCATTATTA CTGAATGTCA GTCATTTCCC CTACTTGATC TGCAATGCCA 3226

ATATCAAGGG CCATGTATCA GGTTTCTGTA TATGTTCCAC TATAATCTTA TGGGACCATG 3286

GTTTTAAATG TGGAATCATT GACAGAAATG TNTTTATGTA GCATATGGCT GTGTATCACT 3346

AGTATATAAT AGAGCAATAT TATGGAGGAA TATGTAGATC CAATCACTTT ACCTATACAA 3406

AATGACTGCT ATGGTGGGAA CACAATAAAC ACCAGTTTTG ACTTTTAAAA AAAAAAAAAA 3466

CAAAAAAAAA AAAAAAAAGC GGCCGCTGAA TTCACCTGCC CGGGCG 3512

SEQ ID NO: 5

Sequence Length: 826

Sequence Type: amino acid

Topology: straight chain

Kind of Sequence: peptide

Sequence:

Met Glu Gly His Thr Asp Met Glu Ile Leu Arg Thr Val Lys Gly Ser

1               5                   10                  15

Ser Thr Gly Glu Val Asn Val His Leu Val Ala Arg Asp Ser Ala Gly

            20                  25                  30

Pro His Pro Gln Leu Pro Thr Thr Ala Phe Ile Ile Pro Thr Asn Ala

        35                  40                  45

Ala Thr Leu Gly Leu Pro Ser Thr Ala Leu Asp Val Pro Tyr Pro Arg

    50                  55                  60

Glu Pro Val His Val Gly Ala Leu Glu Arg Val Ala Gly Ser Glu Pro

65                  70                  75                  80

Val Thr Ala Thr Ile Leu Pro Gln Leu Ser Thr Gly Thr Gly Thr Asn

                85                  90                  95

Ser Thr Val Arg Leu Leu Asp Trp Thr Gly Val Ser Ala Pro Leu Pro

            100                 105                 110

Gly Ser Gly Met Arg Phe Arg Ile Asn Glu Tyr Ala Pro Leu Asn Met

        115                 120                 125

Ile Gly Val Glu Arg Pro Arg Ser Pro Glu Gln Arg His Glu Gly Gly

    130                 135                 140

Met Ala Arg Arg Asp Ala Gly Ile Gln His Pro Asp Val His Gln Asp

145                 150                 155                 160

Arg Gln Asp Ile Thr Cys Leu Glu Pro Pro Val Asp Ala Ser Ser Cys

                165                 170                 175

Lys Cys Gln Ala Cys Gly Pro Gln Gln Ser Ser Gly Leu Asp Val Gly

            180                 185                 190

Ser Ser Gly Asp Arg Cys Ser Gln Pro Phe Gln Lys Arg Ser Val Val

        195                 200                 205

Val Glu Asn Ser Gly Cys Thr Ile Ala Ser Glu Leu Leu Lys Pro Met

    210                 215                 220
Lys Lys Arg Lys His Lys Glu Tyr Gln Ser Pro Ser Glu Glu Ser Glu
225                 230                 235                 240
Pro Glu Ala Val Lys Gln Gly Glu Gly Lys Asp Ala Glu Arg Glu Pro
                245                 250                 255
Thr Pro Ser Thr Pro Glu Asn Glu Glu Trp Ser Arg Ser Gln Leu Val
            260                 265                 270
Ser Ser Glu Lys Lys Asp Gly Trp Ser Trp Glu Ser Tyr Leu Glu Glu
        275                 280                 285
Gln Lys Ala Val Thr Ala Pro Val Ser Leu Phe Gln Asp Ser Gln Ala
    290                 295                 300
Val Thr His Asn Lys Asn Gly Phe Lys Leu Gly Met Lys Leu Glu Gly
305                 310                 315                 320
Ile Asp Pro Gln His Pro Ser Met Tyr Phe Ile Leu Thr Val Ala Glu
                325                 330                 335
Val Cys Gly Tyr Arg Leu Arg Leu His Phe Asp Gly Tyr Ser Glu Cys
            340                 345                 350
His Asp Phe Trp Val Asn Ala Asn Ser Pro Asp Ile His Pro Ala Gly
        355                 360                 365
Trp Phe Glu Lys Thr Gly His Lys Leu Gln Leu Pro Lys Gly Tyr Lys
    370                 375                 380
Glu Glu Glu Phe Ser Trp Ser Gln Tyr Leu Arg Ser Thr Lys Ala Gln
385                 390                 395                 400
Ala Ala Pro Lys His Leu Phe Val Ser Gln Ser His Ser Thr Pro Pro
                405                 410                 415
Val Gly Phe Gln Val Gly Met Lys Leu Glu Ala Val Asp Arg Met Asn

            420                 425                 430
Pro Ser Leu Val Arg Val Ala Ser Val Thr Asp Val Val Asp Ser Arg
        435                 440                 445
Phe Leu Val His Phe Asp Asp Trp Gly Asp Thr Tyr Asp Tyr Trp Cys
    450                 455                 460
Asp Pro Ser Ser Pro Tyr Ile His Pro Val Gly Trp Cys Gln Lys Gln
465                 470                 475                 480
Gly Lys Pro Leu Thr Pro Pro Gln Asp Tyr Pro Asp Pro Asp Ser Phe
                485                 490                 495
Cys Trp Glu Lys Tyr Leu Glu Glu Thr Gly Asp Ser Ala Val Pro Asn
            500                 505                 510
Trp Ala Phe Lys Val Arg Pro Pro His Ser Phe Leu Val Asn Met Lys
        515                 520                 525
Leu Glu Ala Val Asp Arg Arg Asn Pro Ala Leu Ile Arg Val Ala Ser
    530                 535                 540
Val Glu Asp Val Glu Asp His Arg Ile Lys Leu His Phe Asp Gly Trp
545                 550                 555                 560
Ser His Asn Tyr Asp Phe Trp Ile Asp Ala Asp His Pro Asp Ile His
                565                 570                 575
Pro Ala Gly Trp Cys Ser Lys Thr Gly His Pro Leu Glu Pro Pro Leu
            580                 585                 590
Arg Pro Arg Glu Ser Ser Ser Val Ser Pro Gly Gly Cys Pro Pro Leu
        595                 600                 605
Ser His Arg Ser Pro Pro His Thr Lys Thr Ser Gln Tyr Tyr Phe His
    610                 615                 620
His Arg Lys Cys Pro Thr Pro Gly Cys Asp Gly Ser Gly His Val Ser

625                 630                 635                 640
Gly Lys Phe Thr Ala His His Cys Leu Ser Gly Cys Pro Leu Ala Glu
                645                 650                 655
Glu Asn Gln Thr Arg Leu Lys Ala Glu Leu Ser Asp Ser Glu Thr Ala
            660                 665                 670
Ala Arg Lys Lys Asn Pro Phe Asn Leu Ser Pro Arg Lys Lys Leu Arg
        675                 680                 685
His Gln Gly Arg Ile Gly Arg Pro Pro Lys Tyr Arg Lys Met Pro Asp
    690                 695                 700
Glu Asp Phe Gln Ala Leu Pro Pro Ser Val Val His Gln Ser Leu Phe
705                 710                 715                 720
Met Ser Thr Leu Pro Thr His Ala Asp Arg Pro Leu Ser Val Cys Trp
                725                 730                 735
Glu Gln His Cys Lys Leu Leu Pro Gly Val Ala Gly Ile Ser Ala Ser
            740                 745                 750
Ala Val Ser Lys Trp Thr Ile Glu Glu Val Phe Gly Phe Val Gln Thr
        755                 760                 765
Leu Thr Gly Ser Glu Asp Gln Ala Arg Leu Phe Lys Glu Glu Met Ile
    770                 775                 780
Asp Gly Glu Ala Phe Leu Leu Leu Thr Gln Ala Asp Ile Val Lys Ile
785                 790                 795                 800
Met Ser Val Lys Leu Gly Pro Ala Leu Lys Ile Tyr Asn Ala Ile Leu
                805                 810                 815
Met Phe Lys Asn Asn Asp Asp Val Phe Lys
            820                 825

SEQ ID NO: 6
Sequence Length: 2714
Sequence Type: nucleic acid
Strandedness: double
Topology: straight chain
Kind of Sequence: cDNA to mRNA
Sequence:
GGCACGAGGT CGGTCGCTCG GTGCGGGCGG CAACCGCTAG CCATGCAGGT AGGACTCCCT 60
GGAATCATAG ACTAGGTTTA TGAGAGTAGC CTTGTGAAAC CAACAAGATT GGGCAGAGAT 120
GTTGAAATGC TGGCGTAGTC CCAGCAGAGG CCTCAGCCTG AGGAGCAGGA AGCCCTGAAC 180
CTGAGATGGC CCGGAAGAGA GCCCTCAAAT GAAGACACAT CTGG ATG GAG GGG CAC 236
ACT GAC ATG GAG ATA CTA AGG ACA GTG AAA GGG TCT TCC ACA GGG GAG 284
GTC AAC GTG CAC CTG GTG GCC AGA GAC AGC GCA GGT CCC CAC CCT CAG 332
CTG CCT ACA ACC GCC TTC ATT ATT CCT ACC AAT GCA GCT ACG CTT GGT 380
CTG CCT AGC ACT GCC CTG GAT GTG CCC TAT CCC CGG GAG CCA GTC CAC 428
GTG GGT GCC CTG GAG CGG GTG GCT GGC AGC GAA CCC GTA ACA GCT ACT 476
ATT CTG CCA CAG CTA AGC ACT GGA ACG GGG ACC AAT AGC ACA GTC AGG 524
CTT CTG GAT TGG ACT GGC GTT TCT GCA CCT TTA CCT GGT AGC GGC ATG 572
CGG TTC CGG ATA AAC GAG TAC GCG CCA CTG AAC ATG ATA GGA GTG GAG 620
CGA CCA CGA AGC CCG GAG CAG CGA CAT GAG GGT GGC ATG GCC AGG AGA 668
GAT GCG GGC ATC CAG CAC CCT GAT GTT CAC CAG GAC CGT CAA GAT ATA 716
ACT TGT CTG GAA CCC CCG GTA GAT GCG AGT TCC TGC AAG TGC CAG GCT 764
TGT GGG CCT CAA CAA AGT AGT GGC CTA GAC GTT GGT TCA TCG GGA GAC 812
CGA TGC TCT CAG CCT TTC CAG AAG CGC TCA GTC GTA GTA GAG AAC TCA 860
GGC TGT ACC ATC GCT TCT GAG CTC CTG AAA CCC ATG AAA AAG AGG AAA 908
CAT AAG GAG TAT CAG AGC CCA TCA GAG GAG TCA GAG CCG GAG GCT GTG 956

AAG CAA GGA GAA GGA AAA GAC GCA GAG CGA GAG CCC ACC CCT AGC ACC 1004

CCG GAG AAT GAA GAG TGC AGC AGA AGC CAG CTG GTG TCC AGT GAG AAG 1052

AAG GAC GGC TGG TCC TGG GAG TCC TAC CTG GAG GAG CAG AAG GCC GTC 1100

ACA GCC CCA GTT AGC CTC TTC CAG GAC TCC CAG GCA GTC ACC CAT AAC 1148

AAG AAT GGC TTC AAA CTG GGC ATG AAG CTG GAA GGC ATT GAC CCC CAA 1196

CAC CCG TCN ATG TAC TTC ATC CTC ACT GTG GCT GAG GTG TGT GGC TAC 1244

CGC CTG CGC CTG CAC TTT GAT GGC TAC TCT GAG TGC CAC GAC TTC TGG 1292

GTC AAT GCC AAC TCC CCT GAT ATT CAT CCC GCT GGC TGG TTT GAA AAG 1340

ACT GGA CAC AAG CTG CAG CTT CCC AAA GGT TAC AAG GAA GAG GAG TTC 1388

AGC TGG AGC CAG TAC CTG CGC AGC ACG AAA GCT CAG GCA GCC CCC AAG 1436

CAC CTG TTT GTA AGC CAG AGC CAC AGT ACT CCA CCT GTG GGC TTC CAG 1484

GTG GGC ATG AAG CTG GAA GCT GTG GAC CGC ATG AAC CCT TCC CTC GTG 1532

CGC GTG GCC AGT GTG ACC GAT GTG GTG GAC AGT CGC TTC CTG GTG CAC 1580

TTT GAT GAC TGG GGC GAT ACT TAC GAC TAC TGG TGT GAT CCC AGC AGC 1628

CCC TAC ATC CAC CCA GTA GGC TGG TGC CAG AAG CAA GGA AAG CCG CTC 1676

ACC CCT CCA CAA GAC TAC CCA GAC CCT GAC AGC TTC TGC TGG GAG AAA 1724

TAT CTA GAA GAA ACT GGG GAC TCG GCT GTG CCC AAT TGG GCC TTC AAA 1772

GTG CGA CCC CCT CAC AGC TTC CTG GTG AAC ATG AAG CTG GAG GCT GTA 1820

GAC CGC CGG AAC CCA GCA CTG ATC CGG GTA GCC AGT GTG GAA GAT GTG 1868

GAG GAT CAT CGG ATA AAG CTC CAC TTT GAT GGC TGG AGT CAC AAT TAT 1916

GAT TTC TGG ATT GAT GCC GAT CAC CCA GAC ATC CAC CCT GCA GGC TGG 1964

TGC TCC AAG ACA GGA CAT CCC CTG GAG CCT CCT CTC CGG CCC AGA GAG 2012

TCC AGC TCT GTC TCC CCT GGG GGA TGC CCC CCT CTC AGC CAC AGG AGC 2060

CCA CCC CAC ACA AAG ACC TCC CAA TAT TAC TTC CAC CAC CGA AAG TGC 2108

CCC ACT CCT GGC TGT GAC GGG TCC GGC CAT GTC TCT GGC AAG TTC ACA 2156

GCT CAC CAT TGC CTC TCT GGC TGC CCG CTG GCC GAG GAA AAC CAG ACC 2204

CGG CTG AAA GCG GAA CTG TCG CAT TCG GAG ACC GCA GCC AGG AAG AAG 2252

AAC CCG TTT AAT TTG TCC CCA AGG AAA AAG CTT CGC CAT CAA GGC CGG 2300

ATT GGA CGC CCT CCA AAG TAT CGC AAG ATG CCA GAT GAA GAT TTC CAA 2348

GCC CTC CCT CCC AGT GTG GTA CAC CAG TCC CTC TTC ATG TCC ACC TTG 2396

CCG ACC CAC GCT GAT CGC CCA CTG TCT GTG TGC TGG GAG CAA CAC TGC 2444

AAG CTG TTG CCA GGA GTG GCA GGC ATC TCA GCC TCC GCA GTC TCC AAG 2492

TGG ACC ATC GAA GAG GTC TTT GGC TTC GTT CAG ACC TTG ACG GGC TCT 2540

GAG GAC CAA GCG CGC CTC TTC AAA GAG GAG ATG ATT GAC GGC GAG GCC 2588

TTC CTT TTG CTG ACA CAG GCG GAT ATT GTG AAG ATC ATG AGT GTC AAG 2636

CTG GGC CCA GCT TTG AAG ATC TAT AAT GCC ATT CTC ATG TTC AAA AAC 2684

AAT GAC GAT GTC TTT AAG TGA CTGACTGGG 2714

SEQ ID NO: 7

Sequence Length: 826

Sequence Type: amino acid

Topology: straight chain

Kind of Sequence: peptide

Sequence:

Met Glu Gly His Ser Asp Met Glu Ile Ile Arg Ala Val Lys Gly Ser

1               5                   10                  15

Ala Thr Gly Glu Ile Asn Val His Leu Val Ala Arg Asp Ser Ala Gly

            20                  25                  30

Ser His Pro His Leu Pro Thr Thr Thr Phe Ile Ile Pro Thr Asn Ala

        35                  40                  45

Ala Thr Leu Gly Leu Pro Ser Thr Ala Leu Asp Val Ser Tyr Pro Arg

    50                  55                  60

Glu Pro Val [?]is Val Gly Ala Ala Glu Arg Val Ala Gly Ser Glu Pro
65 70 75 80
Val Thr Ala Thr Ile Leu Pro Gln Leu Ser Thr Gly Pro Gly Thr Asn
85 90 95
Ser Thr Val Arg Leu Leu Asp Trp Thr Gly Val Ser Ala Pro Leu Ala
100 105 110
Gly Ser Gly Met Arg Phe Arg Ile Asn Glu Tyr Ala Thr Gln Asn Met
115 120 125
Ile Glu Ile Glu Arg Pro Arg Ser Pro Glu Gln Arg His Glu Gly Gly
130 135 140
Thr Ala Gly Arg Glu Ala Asp Thr Gln His Pro Asp Val His Lys Asp
145 150 155 160
Pro Gln Glu Val Ile Pro Gln Glu Pro Ser Val Asp Ala Gly Ser Cys
165 170 175
Lys Cys Gln Thr Cys Gly Pro Gln Gln Ser Ile Gly Leu Asp Val Gly
180 185 190
Ser Ser Gly Asn Arg Cys Pro Gln Pro Phe Gln Lys Arg Ser Val Ile
195 200 205
Val Glu Asn Ser Gly Cys Thr Val Ala Ser Glu Leu Ile Lys Pro Met
210 215 220
Lys Lys Arg Lys His Lys Glu Tyr Gln Ser Pro Ser Glu Glu Ser Glu
225 230 235 240
Pro Glu Ala Met Lys Gln Gly Lys Gly Lys Asp Pro Asp Arg Glu Pro
245 250 255
Thr Pro Gly Thr Ser Glu Asn Glu Glu Trp Ser Arg Ser Gln Leu Val
260 265 270

Ser Ser Glu Lys Lys Glu Gly Trp Ser Trp Glu Ser Tyr Leu Glu Glu
275 280 285
Gln Lys Ala Val Thr Ala Pro Val Ser Leu Phe Gln Asp Ser Gln Ala
290 295 300
Val Thr His Asn Lys Asn Gly Phe Lys Leu Gly Met Lys Leu Glu Gly
305 310 315 320
Val Asp Pro Gln His Pro Ser Met Tyr Phe Val Leu Thr Val Ala Glu
325 330 335
Val Cys Gly Tyr Arg Leu Arg Leu His Phe Asp Gly Tyr Ser Glu Cys
340 345 350
His Asp Phe Trp Val Asn Ala Asn Ser Pro Asp Ile His Pro Ala Gly
355 360 365
Trp Phe Glu Lys Thr Gly His Lys Leu Gln Pro Pro Lys Gly Cys Lys
370 375 380
Glu Glu Glu Phe Ser Trp Ser Gln Tyr Leu Arg Ser Thr Arg Ala Gln
385 390 395 400
Ala Ala Pro Lys His Leu Phe Val Ser Gln Ser His Ser Pro Pro Pro
405 410 415
Leu Gly Phe Gln Val Gly Met Lys Leu Glu Ala Val Asp Arg Met Asn
420 425 430
Pro Ser Leu Val Cys Val Ala Ser Val Thr Asp Val Val Asp Ser Arg
435 440 445
Phe Leu Val His Phe Asp Asn Trp Asp Asp Thr Tyr Asp Tyr Trp Cys
450 455 460
Asp Pro Ser Ser Pro Tyr Val His Pro Val Gly Trp Cys Gln Lys Gln
465 470 475 480

Gly Lys Pro Leu Thr Pro Pro Gln Asp Tyr Pro Asp Pro Asp Asn Phe
485 490 495
Cys Trp Glu Lys Tyr Leu Glu Glu Thr Gly Ala Ser Ala Val Pro Thr
500 505 510
Trp Ala Phe Lys Val Arg Pro Pro His Ser Phe Leu Val Asn Met Lys
515 520 525
Leu Glu Ala Val Asp Arg Arg Asn Pro Ala Leu Ile Arg Val Ala Ser
530 535 540
Val Glu Asp Val Glu Asp His Arg Ile Lys Ile His Phe Asp Gly Trp
545 550 555 560
Ser His Gly Tyr Asp Phe Trp Ile Asp Ala Asp His Pro Asp Ile His
565 570 575
Pro Ala Gly Trp Cys Ser Lys Thr Gly His Pro Leu Gln Pro Pro Leu
580 585 590
Gly Pro Arg Glu Pro Ser Ser Ala Ser Pro Gly Gly Cys Pro Pro Leu
595 600 605
Ser Tyr Arg Ser Leu Pro His Thr Arg Thr Ser Lys Tyr Ser Phe His
610 615 620
His Arg Lys Cys Pro Thr Pro Gly Cys Asp Gly Ser Gly His Val Thr
625 630 635 640
Gly Lys Phe Thr Ala His His Cys Leu Ser Gly Cys Pro Leu Ala Glu
645 650 655
Arg Asn Gln Ser Arg Leu Lys Ala Glu Leu Ser Asp Ser Glu Thr Thr
660 665 670
Ala Arg Lys Lys Asn Gln Ser Asn Leu Ser Pro Arg Lys Lys Pro Arg
675 680 685

His Gln Gly Arg Ile Gly Arg Pro Pro Lys Tyr Arg Lys Met Pro Asp

690 695 700

Lys Asp Phe Gln Ala Leu Pro Pro Ser Val Val His Gln Ser Leu Phe

705 710 715 720

Met Ser Thr Leu Pro Thr His Ala Asp Arg Pro Leu Ser Val Cys Trp

725 730 735

Glu Gln His Cys Lys Leu Leu Pro Gly Val Ala Gly Ile Ser Ala Ser

740 745 750

Ala Val Ser Lys Trp Thr Ile Glu Glu Val Phe Gly Phe Val Gln Thr

755 760 765

Leu Thr Gly Ser Glu Asp Gln Ala Arg Leu Phe Lys Glu Glu Met Ile

770 775 780

Asp Gly Glu Ala Phe Leu Leu Leu Thr Gln Ala Asp Ile Val Lys Ile

785 790 795 800

Met Ser Val Lys Leu Gly Pro Ala Leu Lys Thr Tyr Asn Ala Ile Leu

805 810 815

Met Phe Lys Asn Asn Asp Asp Val Phe Lys

820 825

SEQ ID NO: 8

Sequence Length: 2922

Sequence Type: nucleic acid

Strandedness: double

Topology: straight chain

Kind of Sequence: cDNA to mRNA

Sequence:

CCCTCAAATT AATACGCAAC CAG ATG GAG GGG CAC AGT GAC ATG GAA ATA ATA 53

AGG GCA GTG AAA GGG TCT GCC ACG GGG GAG ATC AAC GTA CAC CTG GTG 101

GCC AGA GAC AGC GCG GGT TCC CAC CCT CAC CTG CCT ACA ACC ACC TTC 149

ATC ATT CCT ACC AAT GCA GCT ACC CTT GGT CTG CCT AGC ACT GCC CTG 197

GAT GTG TCC TAC CCC CGG GAG CCA GTC CAC GTG GGC GCA GCG GAG CGA 245

GTG GCT GGC AGC GAA CCC GTC ACA GCC ACC ATT CTG CCA CAG CTG AGC 293

ACC GGA CCA GGG ACC AAT AGC ACA GTC AGG CTT CTG GAC TGG ACA GGG 341

GTT TCT GCA CCT TTA GCT GGG AGC GGC ATG AGG TTC CGG ATA AAC GAG 389

TAC GCG ACA CAG AAC ATG ATC GAA ATA GAG CGG CCA CGA AGC CCA GAG 437

CAG CGA CAT GAG GGT GGC ACG GCT GGA AGA GAA GCA GAC ACC CAG CAC 485

CCT GAT GTT CAC AAG GAC CCT CAA GAA GTC ATT CCT CAG GAA CCT TCA 533

GTA GAC GCA GGT TCT TGC AAG TGC CAG ACT TGT GGG CCC CAA CAA AGT 581

ATT GGT CTA GAC GTT GGT TCA TCC GGA AAC CGG TGT CCT CAG CCT TTC 629

CAG AAG CGG TCA GTC ATA GTT GAG AAC TCA GGC TGT ACC GTC GCT TCT 677

GAG CTG ATA AAA CCC ATG AAA AAG AGG AAA CAT AAG GAG TAT CAG AGC 725

CCG TCA GAG GAG TCC GAG CCG GAG GCC ATG AAG CAA GGA AAA GGA AAA 773

GAC CCA GAC AGA GAG CCC ACC CCG GGC ACC TCA GAG AAT GAG GAG TGG 821

AGC AGA AGC CAG CTG GTG TCC AGT GAG AAG AAG GAA GGC TGG TCC TGG 869

GAG TCC TAC CTG GAG GAG CAG AAG GCA GTC ACG GCC CCA GTT AGC CTC 917

TTC CAG GAC TCC CAG GCA GTC ACC CAT AAC AAG AAT GGC TTC AAA CTG 965

GGC ATG AAG CTA GAG GGC GTT GAC CCC CAA CAC CCG TCC ATG TAC TTC 1013

GTC CTC ACT GTG GCT GAG GTA TGT GGC TAT CGC CTA CGC CTG CAC TTT 1061

GAT GGG TAT TCT GAG TGC CAT GAC TTC TGG GTC AAT GCC AAC TCC CCT 1109

GAC ATT CAC CCT GCT GGC TGG TTC GAG AAG ACG GGC CAC AAG CTG CAG 1157

CCT CCC AAA GGT TGC AAG GAG GAG GAG TTC AGC TGG AGC CAG TAC CTG 1205

CGC AGC ACA AGA GCT CAG GCT GCC CCC AAG CAC CTG TTT GTG AGC CAG 1253

AGC CAC AGT CCC CCA CCC CTG GGC TTC CAG GTG GGC ATG AAG CTG GAG 1301

GCT GTT GAC CGC ATG AAC CCG TCC CTT GTC TGC GTG GCC AGT GTG ACC 1349

GAT GTG GTG GAC AGC CGC TTC CTG GTG CAC TTT GAC AAC TGG GAT GAT 1397

ACT TAT GAC TAC TGG TGT GAT CCC AGC AGC CCC TAC GTC CAC CCA GTG 1445

GGC TGG TGC CAG AAG CAA GGA AAG CCC CTC ACC CCT CCA CAA GAC TAC 1493

CCA GAC CCT GAT AAC TTC TGT TGG GAG AAA TAT CTG GAA GAA ACT GGG 1541

GCC TCT GCT GTC CCC ACC TGG GCC TTC AAG GTG CGA CCC CCT CAC AGC 1589

TTC CTG GTC AAT ATG AAG CTG GAG GCT GTG GAC CGC AGG AAC CCA GCC 1637

CTG ATT CGC GTG GCC AGC GTG GAG GAT GTG GAG GAC CAT CGG ATA AAG 1685

ATC CAC TTT GAT GGC TGG AGT CAT GGG TAT GAT TTC TGG ATC GAC GCT 1733

GAC CAC CCA GAC ATC CAC CCT GCC GGC TGG TGC TCC AAG ACA GGA CAT 1781

CCC CTG CAG CCT CCT CTC GGA CCC AGA GAG CCC AGC TCT GCC TCC CCT 1829

GGG GGC TGT CCC CCT CTC AGC TAT AGG AGC CTG CCC CAC ACT AGG ACC 1877

TCC AAA TAC AGC TTT CAC CAC CGG AAG TGC CCC ACT CCT GGT TGC GAC 1925

GGC TCT GGC CAT GTC ACA GGC AAG TTC ACA GCT CAC CAT TGC CTC TCA 1973

GGC TGC CCA CTG GCT GAG AGG AAC CAG AGC CGG CTG AAA GCG GAG CTG 2021

TCC GAC TCA GAG ACC ACA GCC AGG AAG AAG AAC CAG TCT AAT TTG TCC 2069

CCA AGG AAA AAG CCT CGC CAT CAA GGC CGG ATT GGA CGC CCT CCA AAG 2117

TAT CGC AAG ATG CCA GAT AAA GAT TTC CAA GCC CTC CCT CCC AGT GTG 2165

GTA CAC CAG TCC CTC TTC ATG TCC ACC TTG CCG ACC CAC GCT GAT CGC 2213

CCA CTG TCT GTG TGC TGG GAG CAA CAC TGC AAG CTG TTG CCA GGA GTG 2261

GCA GGC ATC TCA GCC TCC GCA GTC TCC AAG TGG ACC ATC GAA GAG GTC 2039

TTT GGC TTC GTT CAG ACC TTG ACG GGC TCT GAG GAC CAA GCG CGC CTC 2357

TTC AAA GAG GAG ATG ATT GAC GGC GAG GCC TTC CTT TTG CTG ACA CAG 2405

GCG GAT ATT GTG AAG ATC ATG AGT GTC AAG CTG GGC CCA GCT TTG AAG 2453

ACC TAT AAT GCC ATT CTC ATG TTC AAA AAC AAT GAC GAT GTC TTT AAG 2501

TGA CTGAC[?]GGGG GCGGGCAG[?]G [?]GGAA[?]GC[?]C CTCCCACCCT CGCTGGGCTC 2554

CCTCTGCAGA [?]GG[?]GC[?][?]C[?] C[?]CCCAA[?][?][?] GAG[?]TCTGCA CCCCTGCCCT CACTGTCTGA 2614

C[?]AGGTGTAG GGGAGCAGAG GAGCCCAGGG AAGAGGGTC[?] AAGTCCTCAG TCCTGGAGTT 2674

CATTTG[?]CTC CAG[?]TGCCCC AGGGAGGG[?]G [?]GACTAAAC[?] GAGGCCTGGC TGACAGAAAG 2734

GGCTGCACG[?] ATCCTGAC[?]A GGGCAGCCAC C[?]TGGTTATC GTCTTAGGAA GTAGAAAGAC 2794

ATGGTAGGCA G[?]CTCTATTC AGACCTATG[?] CCTTCTTGGC ATCTCAATGC CCTTTATCCT 2854

CCTCGGACCC TCTCTTCCTA TTAAGAAG[?]G ACCATGACCT TGGCATACAA AAAAAAAAAA 2914

AAAAAAAA 2922

SEQ ID NO: 9

Sequence Length: 1065

Sequence Type: nucleic acid

Strandedness: double

Topology: straight chain

Kind of Sequence: cDNA to mRNA

Sequence:

ACATAGAGAT AAAGGGAATG AACTATAAAG ACACTGGCAT CTCTGTTTCC CACAAATCAA 60

GACATTCATG AAATTAATGA CTAACTTACA ATGAATTTTT TAGAGGTGTG TCTCATAAGG 120

AAAAACCTGG ATGATATGGG ACCCCGCAGG TGATTGGGGG CGTAGCCTGG AGTGAGGGTT 180

TGGCTGGTGT AGCTTGGAGT GAGGCCCCCT GGCGTGGAGT CTTGAGGCCT GCTGAGGGCG 240

TGAGATGGCA TGAGGCGAAG AGAGGGCCAT GGCACCGACT CCGAGATGGG TCAAGGACCC 300

GTACGGGAGT CGCAATCCTC AGACCCTCCC GCGCTCCAGT TCCGGATAAG CGAGTATAAG 360

CCGCTGAACA TGGCGGGAGT GGAGCAGCCC CCGAGCCCCG AGCTGCGGCA GGAAGGCGTG 420

ACCGAATACG AAGATGGCGG GGCCCCGGCG GGAGATGGCG AGGCGGGCCC CCAACAGGCG 480

GAGGACCACC CCCAGAATCC TCCAGAAGAT CCCAATCAGG ACCCCCCAGA GGATGATAGC 540

ACCTGTCAGT GCCAGGCGTG CGGGCCTCAC CAAGCCGCGG GTCCAGATCT TGGTTCCTCT 600

AATGATGGCT GCCCTCAGCT GTTCCAGGAG CGGTCAGTCA TAGTGGAGAA CTCCTCAGGC 660

TCTACCAGCG CTTCTGAGCT CCTCAAACCC ATGAAGAAGA GGAAGCGCAG GGAATACCAG 720

AGCCCATCAG AGGAGGAGTC GGAGCCAGAG GCCATGGAGA AGCAAGAAGA AGGAAAGGAC 780

CCAGAGGGAC AACCCACTGC TAGCACCCCA GAGAGTGAGG AGTGGAGCAG CAGCCAGCCT 840

GCAACAGGTG AGAAGAAGGA ATGCTGGTCG TGGGAGTCCT ACCTAGAGGA GCAGAAGGCC 900

ATTACTGCTC CAGTCAGCCT CTTCCAGGAC TCCCAGGCAG TCACTCACAA CAAGAATGGC 960

TTCAAACTGG GCATGAAGTT GGAAGGCATT GACCCTCAAC ACCCGTCCAT GTACTTCATC 1020

CTCACCGTGG CTGAGGTATG TGGCTATCGC CTACGCCTGC ACTTT 1065

SEQ ID NO: 10

Sequence Length: 2071

Sequence Type: nucleic acid

Strandedness: double

Topology: straight chain

Kind of Sequence: cDNA to mRNA

Sequence:

CCGTCCCTTG TCTGCGTGGC CAGTGTGACC GATGTGGTGG ACAGCCGCTT CCTGGTGCAC 60

TTTGACAACT GGGATGATAC TTATGACTAC TGGTGTGATC CCAGCAGCCC CTACATCCAC 120

CCAGTGGGCT GGTGCCAGAA GCAAGGAAAG CCCCTCACCC CTCCACAAGA CTACCCAGAC 180

CCTGATAACT TCTGTTGGGA GAAATATCTG GAAGAAACTG GGGCCTCTGC TGTCCCCACC 240

TGGGCCTTCA AGGTGCGACC CCCTCACAGC TTCCTGGTCA ATATGAAGCT GGAGGCTGTG 300

GACCGCAGGA ACCCAGCCCT GATTCGCGTG GCCAGCGTGG AGGATGTGGA GGACCATCGG 360

ATAAAGATCC ACTTTGATGG CTGGAGTCAT GGCTATGATT TCTGGATCGA CGCTGACCAC 420

CCAGACATCC ACCCTGCCGG CTGGTGCTCC AAGACAGGAC ATCCCCTGCA GCCTCCTCTC 480

GGACCCAGAG AGCCCAGCTC TGCCTCCCCT GGGGGCTGTC CCCCTCTCAG CTATAGGAGC 540

CTGCCCCACA CTAGGACCTC CAAATACAGC TTTCACCACC GGAAGTGCCC CACTCCTGGT 600

TGCGACGGCT C[?]GGCCATG[?] CA[?]AGG[?]AAG [?][?]CACAGCTC ACCATTGCCT CTCAGGCTGC 660

CCACTGGCTG AGAGGAACCA GAG[?][?]GGC[?]C AAAGCGGAGC TGTCTGACCC GGAGGCCTCA 720

GCCCGCAAGA AGAACC[?]C[?]C AGGC[?]TC[?]CC CCAAGGAAGA AGCCTCGCCA [?]CACGGCCGA 780

ATTGGACGCC [?][?]CCGAAG[?]A [?]CGAAAGA[?][?] CCGCAGGAAG ATTTCCAGAC CCTCACGCCC 840

GATGTCGTGC ACCAGT[?]CC[?] C[?][?]CATGT[?]A GCCCTGTCGG CCCACCCTGA CCGCTCACTC 900

TCAGTGTGCT GGGAGCAGCA CTGCAAGC[?]C CTGCCAGGAG TAGCGGGCAT CTCAGCCTCG 960

ACAGTCGCCA AGTGGACCA[?] CGATGAGG[?]C TTCGGCTTTG TTCAGACCCT GACAGGTTGT 1020

GAGGACCAAG CACGCCTCTT CAAAGACGAG GCAAGAATAG TCAGAGTGAC CCATGTATCT 1080

GGGAAGACTC TAGTCTGGAC TGTGGCCCAG CTTGGGGACC TTGTGTGCTC AGATCATCTT 1140

CAGGAAGGAA AAGGCATCCT GGAGACAGGA GTCCATTCAC TCCTCTGCTC TCTACCCACT 1200

CATTTGCTTG CCAAACTTAG CTTTGCCAGT GATAGTCAAT ATTAAAGTGT ACTTTTTTCC 1260

CCTTTAATCC AATATAGTTG ATAATTAAAG TGTATTTTGA ATGACACAGA TATTGTGATT 1320

TACTGCAAGG ATCCTAACAC ACACTTAAAA TCAAGAGCCA AGGAGTAGTG AGTTGTAGAT 1380

AAAAAAAGAA TGTCAGCTTT GGAGACAGTC TGGGTTTAAA TCCCAGTTCT GTCAATTTGA 1440

GCTGTTTACT GTCTCTGAGC CTACATCTTC TTGTCTGTAA AATGGAGATA AAATGGGTTT 1500

AATGAGGTCT ACCTTGCAGA GCCATTGTGA GCATTGGAAA TGATGAATGA ATCATACCAG 1560

AACGTCTAGT ATAATTACAG TCATGCATTG CTTAACGATG GGGATACATT CTTAGAAATG 1620

TGTCACTAGG CAATTCTGTC ATTGTGTAAA CATTATAGGA TGTACTTACA CAAACCTAGA 1680

TGTTATATGT ATTTTTATTT ACATGTATAT TTTCACATGA AATACCAAAT GTCACAGCAT 1740

TATTACTGAA TGTCAGTCAT TTCCCCTACT TGATCTGCAA TGCCAATATC AAGGGCCATG 1800

TATCAGGTTT CTGTATATGT TCCACTATAA TCTTATGGGA CCATGGTTTT AAATGTGGAA 1860

TCATTGACAG AAATGTNTTT ATGTAGCATA TGGCTGTGTA TCACTAGTAT ATAATAGAGC 1920

AATATTATGG AGGAATATGT AGATCCAATC ACTTTACCTA TACAAAATGA CTGCTATGGT 1980

GGGAACACAA TAAACACCAG TTTTGACTTT TAAAAAAAAA AAAAACAAAA AAAAAAAAAA 2040

AAAGCGGCCG CTGAATTCAC CTGCCCGGGC G 2071

SEQ ID NO: 11

Sequence Length: 177

Sequence Type: nucleic acid

Strandedness: double

Topology: straight chain

Kind of Sequence: cDNA to mRNA

Sequence:

TTCATCCTCA CTG[?]GGCTGA GGTGTGTGGC TACCGCCTGC GCCTGCACTT TGATGGCTAC 60

TCTGAGTGCC ACGAC[?]TCTG GGTCAATGCC AACTCCCCTG ATATTCATCC CGCTGGCTGG 120

TTTGAAAAGA CTGGACACAA GCTGCAGCTT CCCAAAGGTT ACAAGGAAGA GGAGTTC 177

SEQ ID NO: 12

Sequence Length: 1221

Sequence Type: nucleic acid

Strandedness: double

Topology: straight chain

Kind of Sequence: cDNA to mRNA

Sequence:

GGCACGAGGT CGGTCGCTCG GTGCGGGCGG CAACCGCTAG CCATGCAGGT AGGACTCCCT 60

GGAATCATAG ACTAGGTTTA TGAGAGTAGC CTTGTGAAAC CAACAAGATT GGGCAGAGAT 120

GTTGAAATGC TGGCGTAGTC CCAGCAGAGG CCTCAGCCTG AGGAGCAGGA AGCCCTGAAC 180

CTGAGATGGC CCGGAAGAGA GCCCTCAAAT GAAGACACAT CTGGATGGAG GGGCACACTG 240

ACATGGAGAT ACTAAGGACA GTGAAAGGGT CTTCCACAGG GGAGGTCAAC GTGCACCTGG 300

TGGCCAGAGA CAGCGCAGGT CCCCACCCTC AGCTGCCTAC AACCGCCTTC ATTATTCCTA 360

CCAATGCAGC TACGCTTGGT CTGCCTAGCA CTGCCCTGGA TGTGCCCTAT CCCCGGGAGC 420

CAGTCCACGT GGGTGCCCTG GAGCGGGTGG CTGGCAGCGA ACCCGTAACA GCTACTATTC 480

TGCCACAGCT AAGCACTGGA ACGGGGACCA ATAGCACAGT CAGGCTTCTG GATTGGACTG 540

GCG[?]TTCTGC ACC[?][?]TACC[?] GG[?]AGCGGCA [?]GCGGTTCCG GATAAACGAG TACGCGCCAC 600

TGAACA[?]GA[?] AGGAGTGGAG [?]GA[?]CACGAA [?]CCCGGAGCA GCGACATGAG GGTGGCATGG 660

CCAGGAGAGA TGCGGGCATC CAGCACCCTG ATGTTCACCA GGACCGTCAA GATATAACTT 720

GTCTGGAACC CCCGGTAGA[?] GCGAGT[?]C[?]T GCAAGTGCCA GGCTTGTGGG CCTCAACAAA 780

G[?]AGTGGCC[?] AGACGTTGG[?] [?]CATCGGGAG ACCGATGCTC TCAGCCTTTC CAGAAGCGCT 840

CAGTCG[?]AG[?] AGAGAACTCA GGC[?]GTACCA TCGCTTCTGA GCTCCTGAAA CCCATGAAAA 900

AGAGGAAACA TAAGGAGTA[?] CAGAGCCCAT CAGAGGAGTC AGAGCCGGAG GCTGTGAAGC 960

AAGGAGAAGG AAAAGACGCA GAGCGAGAGC CCACCCCTAG CACCCCGGAG AATGAAGAGT 1020

GGAGCAGAAG CCAGCTGGTG TCCAGTGAGA AGAAGGACGG CTGGTCCTGG GAGTCCTACC 1080

TGGAGGAGCA GAAGGCCGTC ACAGCCCCAG TTAGCCTCTT CCAGGACTCC CAGGCAGTCA 1140

CCCATAACAA GAATGGCTTC AAACTGGGCA TGAAGCTGGA AGGCATTGAC CCCCAACACC 1200

CGTCCATGTA CTTCATCCTC A 1221

SEQ ID NO: 13

Sequence Length: 868

Sequence Type: nucleic acid

Strandedness: double

Topology: straight chain

Kind of Sequence: cDNA to mRNA

Sequence:

CCTGTGGGCT TCCAGGTGGG CATGAAGCTG GAAGCTGTGG ACCGCATGAA CCCTTCCCTC 60

GTGCGCGTGG CCAGTGTGAC CGATGTGGTG GACAGTCGCT TCCTGGTGCA CTTTGATGAC 120

TGGGGCGATA CTTACGACTA CTGGTGTGAT CCCAGCAGCC CCTACATCCA CCCAGTAGGC 180

TGGTGCCAGA AGCAAGGAAA GCCGCTCACC CCTCCACAAG ACTACCCAGA CCCTGACAGC 240

TTCTGCTGGG AGAAATATCT AGAAGAAACT GGGGACTCGG CTGTGCCCAA TTGGGCCTTC 300

AAAGTGCGAC CCCCTCACAG CTTCCTGGTG AACATGAAGC TGGAGGCTGT AGACCGCCGG 360

AACCCAGCAC [?]GA[?]CCGGG[?] AGCCAG[?]GTG GAAGATGTGG AGGATCATCG GATAAAGCTC 420

CACT[?]TGATG GC[?]GGAGTCA CAA[?]TATGA[?] [?]TCTGGATTG ATGCCGATCA CCCAGACATC 480

CACCCTGCAG GCTGGTGCTC CAAGACAGGA CATCCCCTGG AGCCTCCTCT CCGGCCCAGA 540

GAGTCCAGC[?] C[?]G[?]C[?]GCCC [?]GGGGGATGC CCCCCTCTCA GCCACAGGAG CCCACCCCAC 600

ACAAAGACC[?] CC[?]AATA[?][?]A CTTCCACCAC CGAAAGTGCC CCACTCCTGG CTGTGACGGG 660

TCCGGCCATG [?]C[?]C[?]GGCAA GT[?]CACAGCT CACCATTGCC TCTCTGGCTG CCCGCTGGCC 720

GAGGAAAACC AGACCCGGCT GAAAGCGGAA CTGTCGGATT CGGAGACCGC AGCCAGGAAG 780

AAGAACCCGT [?]TAA[?]TTGTC CCCAAGGAAA AAGCTTCGCC ATCAAGGCCG GATTGGACGC 840

CCTCCAAAGT ATCGCAAGA[?] TCCAGAAG 868

SEQ ID NO: 14

Sequence Length: 726

Sequence Type: nucleic acid

Strandedness: double

Topology: straight chain

Kind of Sequence: cDNA to mRNA

Sequence:

GTCCATGTAC TTCGTCCTCA CTGTGGCTGA GGTATGTGGC TATCGCCTAC GCCTGCACTT 60

TGATGGGTAT TCTGAGTGCC ATGACTTCTG GGTCAATGCC AACTCCCCTG ACATTCACCC 120

TGCTGGCTGG TTCGAGAAGA CGGGCCACAA GCTGCAGCCT CCCAAAGGTT GCAAGGAGGA 180

GGAGTTCAGC TGGAGCCAGT ACCTGCGCAG CACAAGAGCT CAGGCTGCCC CCAAGCACCT 240

GTTTGTGAGC CAGAGCCACA GTCCCCCACC CCTGGGCTTC CAGGTGGGCA TGAAGCTGGA 300

GGCTGTTGAC CGCATGAACC CGTCCCTTGT CTGCGTGGCC AGTGTGACCG ATGTGGTGGA 360

CAGCCGCTTC CTGGTGCACT TTGACAACTG GGATGATACT TATGACTACT GGTGTGATCC 420

CAGCAGCCCC TACGTCCACC CAGTGGGCTG GTGCCAGAAG CAAGGAAAGC CCCTCACCCC 480

TCCACAAGAC TACCCAGACC CTGATAACTT CTGTTGGGAG AAATATCTGG AAGAAACTGG 540

GGCCTCTGC[?] GT[?]C[?]CACC[?] GGG[?]CT[?]CAA GGTGCGACCC CCTCACAGCT TCCTGGTCAA 600

[?]A[?]GAAGCT[?] GAGG[?]TG[?]GG ACCGCA[?]GAA [?]CCAGCCC[?]G ATTCGCGTGG CCAGCGTGGA 660

GGATGTGGAG GACCATCGGA TAAAGATCCA CTTTGATGGC TGGAGTCATG GGTATGATTT 720

CTGGA[?] 726

SEQ ID NO: 15

Sequence Length: 1726

Sequence Type: nucleic acid

Strandedness: double

Topology : straight chain

Kind of Sequence : cDNA to mRNA

Sequence:

CCCTCAAATT AATACGCAAC CAGATGGAGG GGCACAGTGA CATGGAAATA ATAAGGGCAG 60

TGAAAGGGTC TGCCACGGGG GAGATCAACG TACACCTGGT GGCCAGAGAC AGCGCGGGTT 120

CCCACCCTCA CCTGCCTACA ACCACCTTCA TCATTCCTAC CAATGCAGCT ACCCTTGGTC 180

TGCCTAGCAC TGCCCTGGAT GTGTCCTACC CCCGGGAGCC AGTCCACGTG GGCGCAGCGG 240

AGCGAGTGGC TGGCAGCGAA CCCGTCACAG CCACCATTCT GCCACAGCTG AGCACCGGAC 300

CAGGGACCAA TAGCACAGTC AGGCTTCTGG ACTGGACAGG GGTTTCTGCA CCTTTAGCTG 360

GGAGCGGCAT GAGGTTCCGG ATAAACGAGT ACGCGACACA GAACATGATC GAAATAGAGC 420

GGCCACGAAG CCCAGAGCAG CGACATGAGG GTGGCACGGC TGGAAGAGAA GCAGACACCC 480

AGCACCCTGA TGTTCACAAG GACCCTCAAG AAGTCATTCC TCAGGAACCT TCAGTAGACG 540

CAGGTTCTTG CAAGTGCCAG ACTTGTGGGC CCCAACAAAG TATTGGTCTA GACGTTGGTT 600

CATCCGGAAA CCGGTGTCCT CAGCCTTTCC AGAAGCGGTC AGTCATAGTT GAGAACTCAG 660

GCTGTACCGT CGCTTCTGAG CTGATAAAAC CCATGAAAAA GAGGAAACAT AAGGAGTATC 720

AGAGCCCGTC AGAGGAGTCC GAGCCGGAGG CCATGAAGCA AGGAAAAGGA AAAGACCCAG 780

ACAGAGAGCC CACCCCGGGC ACCTCAGAGA ATGAGGAGTG GAGCAGAAGC CAGCTGGTGT 840

CCAGTGAGAA GAAGGAAGGC TGGTCCTGGG AGTCCTACCT GGAGGAGCAG AAGGCAGTCA 900

CGGCCCCAGT TAGCCTCTTC CAGGACTCCC AGGCAGTCAC CCATAACAAG AATGGCTTCA 960

AACTGGGCAT GAAGCTAGAG GGCGTTGACC CCCAACACCC GTCCATGTAC TTCGTCCTCA 1020

CTGTGGCTGA GGTATGTGGC TATCGCCTAC GCCTGCACTT TGATGGGTAT TCTGAGTGCC 1080

ATGACTTCTG GGTCAATGCC AACTCCCCTG ACATTCACCC TGCTGGCTGG TTCGAGAAGA 1140

CGGGCCACAA GCTGCAGCCT CCCAAAGGTT GCAAGGAGGA GGAGTTCAGC TGGAGCCAGT 1200

ACCTGCGCAG CACAAGAGCT CAGGCTGCCC CCAAGCACCT GTTTGTGAGC CAGAGCCACA 1260

GTCCCCCACC CCTGGGCTTC CAGGTGGGCA TGAAGCTGGA GGCTGTTGAC CGCATGAACC 1320

CGTCCCTTGT CTGCGTGGCC AGTGTGACCG ATGTGGTGGA CAGCCGCTTC CTGGTGCACT 1380

TTGACAACTG GGATGATACT TATGACTACT GGTGTGATCC CAGCAGCCCC TACGTCCACC 1440

CAGTGGGCTG GTGCCAGAAG CAAGGAAAGC CCCTCACCCC TCCACAAGAC TACCCAGACC 1500

CTGATAACTT CTGTTGGGAG AAATATCTGG AAGAAACTGG GGCCTCTGCT GTCCCCACCT 1560

GGGCCTTCAA GGTGCGACCC CCTCACAGCT TCCTGGTCAA TATGAAGCTG GAGGCTGTGG 1620

ACCGCAGGAA CCCAGCCCTG ATTCGCGTGG CCAGCGTGGA GGATGTGGAG GACCATCGGA 1680

TAAAGATCCA CTTTGATGGC TGGAGTCATG GGTATGATTT CTGGAT 1726

SEQ ID NO: 16

Sequence Length: 1623

Sequence Type: nucleic acid

Strandedness: double

Topology: straight chain

Kind of Sequence: cDNA to mRNA

Sequence:

AGGCTGTTGA CCGCATGAAC CCGTCCCTTG TCTGCGTGGC CAGTGTGACC GATGTGGTGG 60

ACAGCCGCTT CCTGGTGCAC TTTGACAACT GGGATGATAC TTATGACTAC TGGTGTGATC 120

CCAGCAGCCC CTACGTCCAC CCAGTGGGCT GGTGCCAGAA GCAAGGAAAG CCCCTCACCC 180

CTCCACAAGA CTACCCAGAC CCTGATAACT TCTGTTGGGA GAAATATCTG GAAGAAACTC 240
GGGCCTCTGC TGTCCCCACC TGGGCCTTCA AGGTGCGACC CCCTCACAGC TTCCTGGTCA 300
ATATGAAGCT GGAGGCTGTG GACCGCAGGA ACCCAGCCCT GATTCGCGTG GCCAGCGTGG 360
AGGATGTGGA GGACCATCGG ATAAAGATCC ACTTTGATGG CTGGAGTCAT GGGTATGATT 420
TCTGGATCGA CGCTGACCAC CCAGACATCC ACCCTGCCGG CTGGTGCTCC AAGACAGGAC 480
ATCCCCTGCA GCCTCCTCTC GGACCCAGAG AGCCCAGCTC TGCCTCCCCT GGGGGCTGTC 540
CCCCTCTCAG CTATAGGAGC CTGCCCCACA CTAGGACCTC CAAATACAGC TTTCACCACC 600
GGAAGTGCCC CACTCCTGGT TGCGACGGCT CTGGCCATGT CACAGGCAAG TTCACAGCTC 660
ACCATTGCCT CTCAGGCTGC CCACTGGCTG AGAGGAACCA GAGCCGGCTG AAAGCGGAGC 720
TGTCCGACTC AGAGACCACA GCCAGGAAGA AGAACCAGTC TAATTTGTCC CCAAGGAAAA 780
AGCCTCGCCA TCAAGGCCGG ATTGGACGCC CTCCAAAGTA TCGCAAGATG CCAGATAAAG 840
ATTTCCAAGC CCTCCCTCCC AGTGTGGTAC ACCAGTCCCT CTTCATGTCC ACCTTGCCGA 900
CCCACGCTGA TCGCCCACTG TCTGTGTGCT GGGAGCAACA CTGCAAGCTG TTGCCAGGAG 960
TGGCAGGCAT CTCAGCCTCC GCAGTCTCCA AGTGGACCAT CGAAGAGGTC TTTGGCTTCG 1020
TTCAGACCTT GACGGGCTCT GAGGACCAAG CGCGCCTCTT CAAAGAGGAG ATGATTGACG 1080
GCGAGGCCTT CCTTTTGCTG ACACAGGCGG ATATTGTGAA GATCATGAGT GTCAAGCTGG 1140
GCCCAGCTTT GAAGACCTAT AATGCCATTC TCATGTTCAA AAACAATGAC GATGTCTTTA 1200
AGTGACTGAC TGGGGGGGGG CAGTGTGGAA TGCTCCTCCC ACCCTCGCTG GGCTCCCTCT 1260
GCAGATGGTG CTTCTCTCCC AACTTGAGTT CTGCACCCCT GCCCTCACTG TCTGACTAGG 1320
TGTAGGGGAG CAGAGGAGCC CAGGGAAGAG GGTCTAAGTC CTCAGTCCTG GAGTTCATTT 1380
GTCTCCAGTT GCCCCAGGGA GGGTGTGACT AAACTGAGGC CTGGCTGACA GAAAGGGCTG 1440
CACGTATCCT GACTAGGGCA GCCACCTTGG TTATCGTCTT AGGAAGTAGA AAGACATGGT 1500
AGGCAGTCTC TATTCAGACC TATGTCCTTC TTGGCATCTC AATGCCCTTT ATCCTCCTCG 1560
GACCCTCTCT TCCTATTAAG AAGTGACCAT GACCTTGGCA TACAAAAAAA AAAAAAAAAA 1620
AAA 1623
SEQ ID NO: 17

Sequence Length: 20
Sequence Type: amino acid
Topology: straight chain
Kind of Sequence: peptide
Sequence:
Cys Gly Arg Ile Gly Arg Pro Pro Lys Tyr Arg Lys Ile Pro Gln Glu
1               5                   10                  15
Asp Phe Gln Thr
            20

SEQ ID NO: 18
Sequence Length: 21
Sequence Type: amino acid
Topology: straight chain
Kind of Sequence: peptide
Sequence:
Cys Met Lys Lys Arg Lys Arg Arg Glu Tyr Gln Ser Pro Ser Glu Glu
1               5                   10                  15
Glu Ser Glu Pro Glu
            20

SEQ ID NO: 19
Sequence Length: 21
Sequence Type: amino acid
Topology: straight chain
Kind of Sequence: peptide

Sequence:

Cys Met Lys Lys Arg Lys Arg Arg Glu Tyr Gln Ser Pro Ser Glu Glu
1               5                   10                  15
Glu Ser Glu Pro Glu
            20

SEQ ID NO: 20
Sequence Length: 21
Sequence Type: nucleic acid
Strandedness: single
Topology: straight chain
Kind of Sequence: another nucleic acid
Sequence:
CCATCAAAGT GCAGGCGTAG G 21

SEQ ID NO: 21
Sequence Length: 21
Sequence Type: nucleic acid
Strandedness: single
Topology: straight chain
Kind of Sequence: another nucleic acid
Sequence:
TAGCCACATA CCTCAGCCAC G 21

SEQ ID NO: 22

Sequence Length: 21
Sequence Type: nucleic acid
Strandedness: single
Topology: straight chain
Kind of Sequence: another nucleic acid
Sequence:
AAGCTGTTTG ACCGCATGAA C 21

SEQ ID NO: 23
Sequence Length: 21
Sequence Type: nucleic acid
Strandedness: single
Topology: straight chain
Kind of Sequence: another nucleic acid
Sequence:
AAGCACCTGT TTGTGAGCCA G 21

SEQ ID NO: 24
Sequence Length: 22
Sequence Type: nucleic acid
Strandedness: single
Topology: straight chain
Kind of Sequence: another nucleic acid
Sequence:
ATGAGGCGAA GAGAGGGCCA TG 22
SEQ ID NO: 25

Sequence Length: 24
Sequence Type: nucleic acid
Strandedness: single
Topology: straight chain
Kind of Sequence· another nucleic acid
Sequence:
GTTAGGATCC TTGCAGTAAA TCAC 24

SEQ ID NO: 26
Sequence Length: 23
Sequence Type: nucleic acid
Strandedness: single
Topology: straight chain
Kind of Sequence: another nucleic acid
Sequence:
TGAACTCCTC CTCCTTGTAA CCT 23

SEQ ID NO: 27
Sequence Length: 21
Sequence Type: nucleic acid
Strandedness: single
Topology: straight chain
Kind of Sequence: another nucleic acid
Sequence:
GTCCATGTAC TTCATCCTCA C 21
SEQ ID NO: 28

Sequence Length: 17
Sequence Type: nucleic acid
Strandedness: single
Topology: straight chain
Kind of Sequence: another nucleic acid
Sequence:
CAGGAAACAG CTATGAC 17

SEQ ID NO: 29
Sequence Length: 20
Sequence Type: nucleic acid
Strandedness: single
Topology: straight chain
Kind of Sequence: another nucleic acid
Sequence:
AATTAACCCT CACTAAAGGG 20

SEQ ID NO: 30
Sequence Length: 20
Sequence Type: nucleic acid
Strandedness: single
Topology: straight chain
Kind of Sequence: another nucleic acid
Sequence:
GTTGAAATGC TGGCGTAGTC 20
SEQ ID NO: 31

Sequence Length: 24
Sequence Type: nucleic acid
Strandedness: single
Topology: straight chain
Kind of Sequence: another nucleic acid
Sequence:
CCCAGTCAGT CACTTAAAGA CATC 24

SEQ ID NO: 32
Sequence Length: 23
Sequence Type: nucleic acid
Strandedness: single
Topology: straight chain
Kind of Sequence: another nucleic acid
Sequence:
ATCCAGAAAT CATAGCCATG ACT 23

SEQ ID NO: 33
Sequence Length: 21
Sequence Type: nucleic acid
Strandedness: single
Topology: straight chain
Kind of Sequence: another nucleic acid
Sequence:
GTTGAAATGC TGGCGTAGTC C 21
SEQ ID NO: 34

Sequence Length: 20
Sequence Type: nucleic acid
Strandedness: single
Topology: straight chain
Kind of Sequence: another nucleic acid
Sequence:
GGCCACGCGT CGACTAGTAC 20

## Claims

1. A protein comprising an amino acid sequence having at least an amino acid sequence set forth in any one of SEQ ID NO: 1, 3, 5 and 7 of the Sequence Listing.

2. A protein comprising an amino acid sequence wherein one or more amino acids of the amino acid sequence set forth in SEQ ID NO: 1, 3, 5 or 7 of the Sequence Listing are substituted, deleted or added.

3. A polynucleotide encoding the protein according to claim 1 or 2.

4. An antisense polynucleotide having a base sequence of an antisense strand of the polynucleotide according to claim 3, and a derivative of said antisense polynucleotide.

5. A polynucleotide having 12 or more consecutive bases which are a part of the polynucleotide according to claim 3 or a part of the antisense polynucleotide according to claim 4.

6. A polynucleotide according to any one of claims 3 to 5, wherein said polynucleotide is chemically modified.

7. A method for acquiring a cDNA homologous to a DNA having a base sequence set forth in any one of SEQ ID NO: 2, 4, 6 and 8 of the Sequence Listing, said method using the polynucleotide according to any of claims 4 to 6 as a probe to acquire from a cDNA library, the cDNA hybridizing with said polynucleotide serving as the probe.

8. A method for acquiring the cDNA according to claim 7, wherein the polynucleotide used as the probe is a DNA having a base sequence comprising the part from the 865th base T to the 1809th base C of the base sequence set forth in SEQ ID NO: 2 of the Sequence Listing, a DNA having a base sequence comprising the part from the 1062nd base T to the 2006th base C of the base sequence set forth in SEQ ID NO: 6 of the Sequence Listing, or a DNA having a base sequence comprising the part from the 861st base T to the 1805th base C of the base sequence set forth in SEQ ID NO: 8 of the Sequence Listing.

9. A cDNA acquired by the method according to claim 7 or 8, said cDNA being homologous to the DNA having a base sequence set forth in any one of SEQ ID NO: 2, 4, 6 and 8 of the Sequence Listing.

10. A protein homologous to the protein according to claim 1 and comprising an amino acid sequence encoded by the cDNA according to claim 9.

11. An antibody recognizing the protein according to any one of claims 1, 2 and 10.
